(19) Europäisches Patentamt
European Patent Office
Office européen des brevets

(11) EP 3 340 989 B1

(12) EUROPEAN PATENT SPECIFICATION

(45) Date of publication and mention of the grant of the patent:
**16.08.2023 Bulletin 2023/33**

(21) Application number: **16760803.3**

(22) Date of filing: **24.08.2016**

(51) International Patent Classification (IPC):
***A61K 31/496*** (2006.01) ***A61K 31/506*** (2006.01)
***A61K 31/519*** (2006.01) ***A61K 31/5375*** (2006.01)
***A61P 35/00*** (2006.01)

(52) Cooperative Patent Classification (CPC):
(C-Sets available)
**A61K 31/506; A61K 31/337; A61K 31/4184; A61K 31/4412; A61K 31/4439; A61K 31/496; A61K 31/517; A61K 31/519; A61K 31/5375; A61K 31/5377; A61K 31/635; A61P 35/00** (Cont.)

(86) International application number:
**PCT/IB2016/055050**

(87) International publication number:
**WO 2017/037579 (09.03.2017 Gazette 2017/10)**

(54) **MDM2 INHIBITORS AND COMBINATIONS THEREOF**

MDM2-INHIBITOREN UND VERWENDUNGEN DAFÜR

INHIBITEURS DE MDM2 ET COMBINAISONS DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.08.2015 US 201562211080 P**
**19.10.2015 US 201562243337 P**
**04.11.2015 US 201562250574 P**

(43) Date of publication of application:
**04.07.2018 Bulletin 2018/27**

(60) Divisional application:
**23177095.9**

(73) Proprietor: **Novartis AG**
**4056 Basel (CH)**

(72) Inventors:
- **HALILOVIC, Ensar**
  **Cambridge, Massachusetts 02139 (US)**
- **CAPONIGRO, Giordano**
  **Cambridge, Massachusetts 02139 (US)**
- **HORN-SPIROHN, Thomas**
  **Cambridge, Massachusetts 02139 (US)**
- **LEHAR, Joseph**
  **Cambridge, Massachusetts 02139 (US)**

(74) Representative: **Petersen, Holger**
**Novartis Pharma AG**
**Lichtstrasse 35**
**4056 Basel (CH)**

(56) References cited:
**EP-A1- 2 752 191** **WO-A1-2015/084804**
**WO-A2-2015/070224** **US-A1- 2013 281 473**

- **KOJIMA K ET AL: "Concomitant inhibition of MDM2 and Bcl-2 protein function synergistically induce mitochondrial apoptosis in AML", CELL CYCLE, LANDES BIOSCIENCE, US, vol. 5, no. 23, 1 December 2006 (2006-12-01), pages 2778-2786, XP002740450, ISSN: 1551-4005, DOI: 10.4161/CC.5.23.3520 [retrieved on 2006-12-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 340 989 B1

- **RONGQING PAN ET AL: "Activation of p53 By Novel MDM2 Antagonist RG7388 Overcomes AML Inherent and Acquired Resistance to Bcl-2 Inhibitor ABT-199 (GDC-0199)", BLOOD; 56TH ASH ANNUAL MEETING AND EXPOSITION; SAN FRANCISCO, CA; DECEMBER 6-9, 2014, AMERICAN SOCIETY OF HEMATOLOGY, US, [Online] vol. 124, 1 December 2014 (2014-12-01), XP002759670, ISSN: 0006-4971 Retrieved from the Internet: URL:www.bloodjournal.org/content/124/21/2162> [retrieved on 2016-07-08]**
- **E. WEISBERG ET AL: "Inhibition of Wild-Type p53-Expressing AML by the Novel Small Molecule HDM2 Inhibitor CGM097", MOLECULAR CANCER THERAPEUTICS, vol. 14, no. 10, 1 October 2015 (2015-10-01), pages 2249-2259, XP055309846, US ISSN: 1535-7163, DOI: 10.1158/1535-7163.MCT-15-0429**

(52) Cooperative Patent Classification (CPC): (Cont.)

C-Sets
**A61K 31/496, A61K 2300/00;**
**A61K 31/506, A61K 2300/00;**
**A61K 31/519, A61K 2300/00;**
**A61K 31/5375, A61K 2300/00**

## Description

FIELD OF THE DISCLOSURE

The present invention relates to a pharmaceutical combination comprising the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one, or a pharmaceutically acceptable salt thereof, and the Bcl2 inhibitor ABT-199, or a pharmaceutically acceptable salt thereof, particularly for use in the treatment of a cancer. The invention is defined in the claims.

BACKGROUND OF THE DISCLOSURE

The advent of targeted therapies for cancer has increased patient lifespan for various malignancies and helped to appreciate the complexity of tumors through the study of drug resistance mechanisms. The fact that clinical responses to targeted agents are generally incomplete and/or transient results from a multitude of factors that can be broadly put into two classes: toxicities that prevent optimal dosing of drugs and consequently limit target engagement (Brana and Siu 2012, Chapman, Solit et al. 2014), and the ability of cancers to adapt and maintain their proliferative potential against perturbations (Druker 2008, Chandarlapaty 2012, Doebele, Pilling et al. 2012, Duncan, Whittle et al. 2012, Katayama, Shaw et al. 2012, Lito, Rosen et al. 2013, Sullivan and Flaherty 2013, Solit and Rosen 2014). Combinations of drugs can address both these factors by improving overall efficacies and at the same time targeting tumor robustness and complexity to counter resistance (Robert, Karaszewska et al. 2015, Turner, Ro et al. 2015). It is not yet clear how many drugs are required and which processes need to be targeted in combination to overcome cancer. But it is almost certain that different pathways or drivers need to be inhibited, most likely requiring two or more drugs (Bozic, Reiter et al. 2013). This is supported by the successes of combining conventional chemotherapeutic agents to treat cancers (DeVita 1975), and combination therapies for infectious diseases such as HIV (Porter, Babiker et al. 2003), as well as by theoretic approaches showing how biological robustness can be challenged by increasing the order of perturbations (Lehar, Krueger et al. 2008).

K Kojima et al "Cell cycle, Landes Bioscience, US, vol. 5, no. 23, 1 December 2006, pages 2778-2786, XP002740450, references that concomitant inhibition of MDM2 and Bcl-2 protein function synergistically induce mitochondrial apoptosis in AML.

In spite of numerous treatment options for patients with specific types of cancer, there remains a need for effective and safe combination therapies that can be administered for the effective long-term treatment of cancer.

SUMMARY OF THE DISCLOSURE

It is an object of the present disclosure to provide for a medicament to improve treatment of a cancer, in particular to improve treatment of cancer through inhibition of cell growth (proliferation) and induction of apoptosis. It is an object of the present disclosure to find novel combination therapies, which selectively synergize in inhibiting proliferation and/or in inducing apoptosis.

Such inhibitors as MDM2 inhibitors, MEK inhibitors and BCL2 inhibitors, as a monotherapy, demonstrate anti-proliferative (cytostatic) and pro-apoptotic (cytotoxic) activities in vitro and in vivo pre-clinical assays. Surprisingly it has been found that a pharmaceutical combination comprising

the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one, or a pharmaceutically acceptable salt thereof, and the Bcl2 inhibitor ABT-199, or a pharmaceutically acceptable salt thereofhas a beneficial synergistic interaction, improved anti-cancer activity, improved anti-proliferative effect, and improved pro-apoptotic effect. These combinations demonstrated a synergistic effect in cell growth inhibition and induction of cell death by apoptosis.

Further, it has been found that this combination may advantageously comprise further inhibitors selected from EGFR inhibitors, PI3K inhibitors and BRAF inhibitors. In addition, CDK4/6 inhibitor or standard of care such as paclitaxel can be added to the combination, which can lead to further synergistic effect or strong induction of apoptosis. A combination of the MDM2 inhibitor of the invention with a Bcl2 inhibitor of the invention can be supplemented by a BRAF inhibitor (e.g. dabrafenib) and CMET inhibitor (e.g. PF-04217903) to form a quadruple combination. The latter combination was found to be weakly synergistic, but with strongly inducing apoptosis.

In another aspect, the present disclosure relates to a pharmaceutical composition comprising the pharmaceutical combination of the disclosure and at least one pharmaceutically acceptable carrier.

In one aspect, the present disclosure relates to the pharmaceutical combination or the pharmaceutical composition of the disclosure for use as a medicine.

In another aspect, the present disclosure relates to the pharmaceutical combination or the pharmaceutical

composition of the disclosure for use in the treatment of cancer.

Specifically, the present disclosure provides the following aspects, advantageous features and specific embodiments, respectively alone or in combination, as listed in the claims below.

BRIEF DESCRIPTION OF THE DRAWINGS

**FIG. 1** Dose-response curves for 8 TP53 wild-type colorectal cancer cell lines for the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino} -phenyl)-1,4-dihydro-2H-isoquinolin-3-one (COMPOUND A) (circle) and the MEK inhibitor trametinib (triangle) and their combination (diamond). The x-axis indicates the log10 of the treatment dilution; the y-axis indicates the cell count after treatment relative to DMSO. Combinations result from a fixed-ratio (1: 1) combination of the single agents. The strong dashed line indicated the number of cells before the start of the treatment ('baseline').

**FIG. 2** Dose-response curves for 8 TP53 wild-type colorectal cancer cell lines for the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one (COMPOUND B) (circle) and the MEK inhibitor trametinib (triangle) and their combination (diamond). The x-axis indicates the log10 of the treatment dilution; the y-axis indicates the cell count after treatment relative to DMSO. Combinations result from a fixed-ratio (1: 1) combination of the single agents. The strong dashed line indicated the number of cells before the start of the treatment ('baseline').

**FIG. 3** Isobologram analysis at the 75% inhibition level for combinations of the MDM2 inhibitor (S)-1-(4-Chloro-phenyl)-7-isopropoxy-6-methoxy-2-(4-fmethyl-[4-(4-methyl-3-oxo-piperazin-1-yl)-trans-cyclohexylmethyl]-amino} -phenyl)-1,4-dihydro-2H-isoquinolin-3-one (COMPOUND A) or the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one (COMPOUND B) (y-axis) with the MEK inhibitor trametinib (x-axis) over 8 TP53 wild-type colorectal cancer cell lines. Points on the diagonal curve indicate an additive effect, points to the right of it an antagonism, and points to the left of it synergy. The hollow circle shows the combination with the lowest combinations index (strongest synergy) (see Table 2 for the value).

**FIG. 4** Maximum Caspase 3/7 induction for the MDM2 inhibitor (S)-1-(4-Chlorophenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib and their combination in 5 TP53 wild-type colorectal cancer cell lines and after 24h, 48h, and 72h (different shades of grey). The x-axis indicates the treatment; the y-axis indicates the maximum Caspase 3/7 induction (% of cells) seen for each treatment.

**FIG. 5** Long-term colony formation assays for single agents and combination of the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino} -phenyl)-1,4-dihydro-2H-isoquinolin-3-one (COMPOUND A) and the MEK inhibitor trametinib. "COMPOUND A (L)": 0.33$\mu$M; "COMPOUND A (H)": 1$\mu$M; "trametinib (L)" for all but LIM2405 and SW48: 4nM; "trametinib (H)" for all but LIM2405 and SW48: 12nM; "trametinib (L)" for LIM2405 and SW48: 1nM, "trametinib (H)" for LIM2405 and SW48: 3nM. (A) Representative images of cells after crystal violet staining. (B) Quantification of crystal violet signal from (A). Bars show average $\pm$ standard deviation for n=3 replicates. For significance test see Table 3. RFU = relative fluorescence unit.

**FIG. 6** FACS analysis for the MDM2 inhibitor (S)-1-(4-Chloro-phenyl)-7-isopropoxy-6-methoxy-2-(4- f methyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib and their combination after 24h treatment. "COMPOUND A (L)": 0.33$\mu$M; "COMPOUND A (H)": 1$\mu$M; "trametinib (L)" for all but LIM2405 and SW48: 4nM; "trametinib (H)" for all but LIM2405 and SW48: 12nM; "trametinib (L)" for LIM2405 and SW48: 1nM, "trametinib (H)" for LIM2405 and SW48: 3nM. The stacked bars indicate the percentage of the cell population in each of the cell cycle phases: subG1, G1, S, and G2.

**FIG. 7** Western blot analysis of the MDM2 inhibitor (S)-1-(4-Chloro-phenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib and their combination after 24h treatment. "COMPOUND A (L)": 0.33$\mu$M; "COMPOUND A (H)": 1$\mu$M; "trametinib (L)" for all but LIM2405 and SW48: 4nM; "trametinib (H)" for all but LIM2405 and SW48: 12nM; "trametinib (L)" for LIM2405 and SW48: 1nM, "trametinib (H)" for LIM2405 and SW48:

3nM.

**FIG. 8** qRT-PCR analysis of 5 target genes for of the MDM2 inhibitor (S)-1-(4-Chlorophenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib and their combination after 10h treatment. "COMPOUND A (L)": 0.33μM; "COMPOUND A (H)": 1μM; "trametinib (L)" for all but LIM2405 and SW48: 4nM; "trametinib (H)" for all but LIM2405 and SW48: 12nM; "trametinib (L)" for LIM2405 and SW48: 1nM, "trametinib (H)" for LIM2405 and SW48: 3nM. Bars show differential expression on log2 scale compared to DMSO treatment, error bars show standard deviation for n=2 replicates.

**FIG. 9** Dose-response curves for the MDM2 inhibitor (S)-1-(4-Chloro-phenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A) (circles), the MEK inhibitor trametinib (COMPOUND B, triangles), the BCL-2/-XL inhibitor navitoclax (ABT-263) (COMPOUND C, diamonds), and their combinations A+B (circles, dotted line), A+C (triangles), B+C (diamonds) and A+B+C (circles, full line) over 5 TP53 wild type colorectal cancer cell lines. The x-axis indicates the log10 of the treatment dilution; the y-axis indicates the cell count after treatment relative to DMSO. The strong dashed line indicated the number of cells before the start of the treatment ('baseline').

**FIG. 10** Maximum Caspase 3/7 induction for the MDM2 inhibitor (S)-1-(4-Chlorophenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib (B), and the BCL-2/-XL inhibitor navitoclax (ABT-263) (C), and their combinations A+B, A+C, B+C, and A+B+C in 5 TP53 wild type colorectal cancer cell lines and after 24h, 48h, and 72h (different shades of grey). The x-axis indicates the treatment; the y-axis indicates the maximum Caspase 3/7 induction (% of cells) seen for each treatment.

**FIG. 11** Dose-response curves for the MDM2 inhibitor (S)-1-(4-Chloro-phenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A) (circles), the MEK inhibitor trametinib (COMPOUND B, triangles), the EGFR inhibitor erlotinib (COMPOUND C, diamonds), and their combinations A+B (circles, dotted line), A+C (triangles), B+C (diamonds) and A+B+C (circles, full line) over 5 TP53 wild type colorectal cancer cell lines. The x-axis indicates the log10 of the treatment dilution; the y-axis indicates the cell count after treatment relative to DMSO. The strong dashed line indicated the number of cells before the start of the treatment ('baseline').

**FIG. 12** Maximum Caspase 3/7 induction for the MDM2 inhibitor (S)-1-(4-Chlorophenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib (COMPOUND B), and the EGFR inhibitor erlotinib (COMPOUND C), and their combinations A+B, A+C, B+C, and A+B+C in 5 TP53 wild type colorectal cancer cell lines and after 24h, 48h, and 72h (different shades of grey). The x-axis indicates the treatment; the y-axis indicates the maximum Caspase 3/7 induction (% of cells) seen for each treatment.

**FIG. 13** Dose-response curves for the PIK3CA inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND A) (circles), the MDM2 inhibitor (S)-1-(4-Chloro-phenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND B) (triangles), the BCL-2/-XL inhibitor navitoclax (ABT-263) (COMPOUND C) (diamonds), and their combinations A+B (circles, dotted line), A+C (triangles), B+C (diamonds) and A+B+C (circles, full line) over 5 TP53 wild type colorectal cancer cell lines. The x-axis indicates the log10 of the treatment dilution; the y-axis indicates the cell count after treatment relative to DMSO. The strong dashed line indicated the number of cells before the start of the treatment ('baseline').

**FIG. 14** Maximum Caspase 3/7 induction for the PIK3CA inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND A), the MDM2 inhibitor (S)-1-(4-Chlorophenyl)-7-isopropoxy-6-methoxy-2-(4-fmethyl-[4-(4-methyl-3-oxo-piperazin-1-yl)-trans-cyclohexylmethyl]-amino}-phenyl)-1,4-dihydro-2H-isoquinolin-3-one (COMPOUND B), the BCL-2/-XL inhibitor navitoclax (ABT-263) (COMPOUND C), A+B, A+C, B+C, and A+B+C in 5 TP53 wild type colorectal cancer cell lines and after 24h, 48h, and 72h (different shades of grey). The x-axis indicates the treatment; the y-axis indicates the maximum Caspase 3/7 induction (% of cells) seen for each treatment.

**FIG. 15** Dose-response curves for the MDM2 inhibitor (S)-1-(4-Chloro-phenyl)-7-isopropoxy-6-methoxy-2-(4- f methyl-[4-(4-methyl-3-oxo-piperazin-1-yl)-trans-cyclohexylmethyl]-amino}-phenyl)-1,4-dihydro-2H-isoquinolin-3-one (COMPOUND A) (circle) and the BCL-2/-XL inhibitor navitoclax (ABT-263) (triangle) and the combination of COMPOUND A and ABT-263 (diamond) over 5 TP53 wild-type colorectal cancer cell lines. The x-axis indicates the log10 of the treatment dilution; the y-axis indicates the cell count after treatment relative to DMSO. The strong dashed line indicated the number of cells before the start of the treatment ('baseline').

**FIG. 16** Maximum Caspase 3/7 induction for COMPOUND A and ABT-263 and the combination of the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A) and the BCL-2/-XL inhibitor navitoclax (ABT-263) in 5 TP53 wild-type colorectal cancer cell lines and after 24h, 48h, and 72h (different shades of grey). The x-axis indicates the treatment; the y-axis indicates the maximum Caspase 3/7 induction (% of cells) seen for each treatment.

**FIG. 17** KRAS mutant HCT-116 xenografts were treated with the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A), the MEK inhibitor trametinib (COMPOUND B), and the BCL-2/-XL inhibitor ABT-263 (COMPOUND C), or combinations thereof. Specifically, the xenografts were treated with vehicle (G1), ABT-263 (G2, 100mg/kg daily), COMPOUND A (G3, 100mg/kg three times weekly), trametinib (G4, 0.3mg/kg daily), the combination of COMPOUND A and trametinib (G5), or the combination of all three agents (G6). At day 9 ABT-263 was added to G3-G5. The mean percentage change in tumor volume relative to the initial tumor volume is shown. Error bars represent SEM.

**FIG. 18** Waterfall plots showing the percent change in tumor volume (relative to initial volume) for individual tumors in the cohorts G3-G6 (as described in example 10 and Figure 17) following 9 days of treatment (A), and 19 days of treatment (10 days after sequential addition of ABT-263)(B).

DETAILED DESCRIPTION OF THE DISCLOSURE

In one embodiment, the present invention relates to a pharmaceutical combination comprising the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one, or a pharmaceutically acceptable salt thereof, and the Bcl2 inhibitor ABT-199, or a pharmaceutically acceptable salt thereof. It has been determined that the combination could be used to efficiently treat cancer. In particularly, it has been determined that the combination could be used to efficiently treat cancer due to a synergistic effect in inhibition of cell proliferation and / or induction of apoptosis. Accordingly, the combinations of the present disclosure, in particular triple and further combination, may shift a "cytostatic" response to a "cytotoxic" response, thus achieving cancer regression.

The terms "a" and "an" and "the" and similar references in the context of describing the disclosure (especially in the context of the following claims) are to be construed to cover both the singular and the plural, unless otherwise indicated herein or clearly contradicted by context. Where the plural form is used for compounds, patients, cancers and the like, this is taken to mean also a single compound, patient, or the like.

The term "synergistic effect" as used herein refers to action of two or three therapeutic agents such as, for example, a compound of formula (I), e.g., Compound A, and at least one MEK inhibitor compound of the present disclosure, e.g., Compound A, and at least one BCL2 inhibitor compound of the present disclosure, producing an effect, for example, slowing the progression of a proliferative disease, particularly cancer, or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of a drug combination. The corresponding graphs associated with the equations referred to above are the concentration-effect curve, isobologram curve and combination index curve, respectively.

In particular, it has been demonstrated that combined inhibition of MDM2 and MEK in TP53 wild-type colorectal cancer provides an improved (Example 1, Figures 1 and 2, Table 2) and more durable response (Example 1, Figure 5, Table 3) compared to each single agents. Also, combined inhibition of MDM2 and Bcl2 in TP53 wild-type colorectal cancer showed stronger induction of apoptosis compared to the single agents (Example 5, Figure 16). Even further, a triple combination of a MDM2 inhibitor, a MEK inhibitor and a Bcl2 inhibitor caused synergistic inhibition over the drug pairs in 2/5 TP53 wild-type colorectal cancer cell models tested (Example 2, Table 5), and in four of those cell lines the triple combination showed stronger apoptosis compared to the pair wise combinations (Example 2, Figure 10). Thus,

the combinations of the present disclosure provide an effective therapy option capable of improving responses compared to each of the single agents and can lead to more durable responses in the clinic.

The term "MDM2 inhibitor" or "HDM2 inhibitor" or "Mdm2 inhibitor" as used herein, refer to any compound inhibiting the HDM2/p53 (Mdm2/p53) interaction association. HDM2 (Human homolog of murine double minute 2) is a negative regulator of p53. Mdm2 inhibitors are useful in pharmaceutical compositions for human or veterinary use where inhibition of Mdm2/p53 association is indicated, e.g., in the treatment of tumors and/or cancerous cell growth. In particular, Mdm2 inhibitors are useful in the treatment of human cancer, since the progression of these cancers may be at least partially dependent upon overriding the "gatekeeper" function of p53, for example the overexpression of Mdm2.

According to the present invention, the Mdm2 inhibitor is (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one, or a pharmaceutically acceptable salt thereof The Mdm2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one belongs to a novel class of imidazopyrrolidinone compounds, and shows potent inhibition of the MDM2/p53 interaction (this term including in particular Hdm2/p53 interaction). In particular, this compound acts as an inhibitor of MDM2 interaction with p53 by binding to MDM2. The MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one, which is the Mdm2i inhibitor of the invention, is a compound of formula I, and described in Example 102 of WO2013/111105:

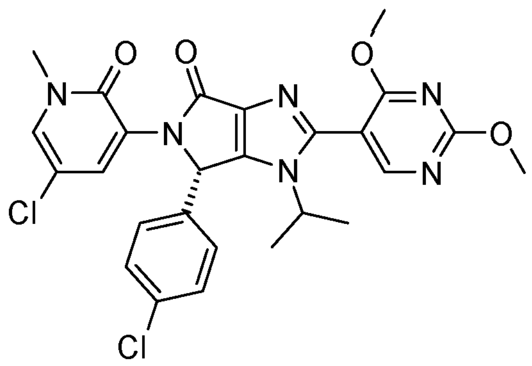

(I).

The crystalline forms of (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one are described as EX6, EX7 and EX8 in WO2013/111105. The disclosure encompasses succinic acid co-crystal of the (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one compound. The compound can be also be in a form of an ethanol solvate.

The term "a MEK inhibitor" is defined herein to refer to a compound which targets, decreases or inhibits the kinase activity of MAP kinase, MEK. A target of a MEK inhibitor includes, but is not limited to, ERK. An indirect target of a MEK inhibitor includes, but is not limited to, cyclin D1.

Pharmaceutical combinations of the present disclosure can include at least one MEK inhibitor compound selected from the group consisting of trametinib, 6-(4-bromo-2-fluorophenylamino)-7-fluoro-3-methyl-3H-benzoimidazole-5-carboxylic acid (2-hydroxyethoxy)-amide, (S)-5-fluoro-2-(2-fluoro-4-(methylthio)phenylamino)-N-(2-hydroxypropoxy)-1-methyl-6-oxo-1,6-dihydropyridine-3-carboxamide, PD0325901, PD-184352, RDEA119, XL518, AS-701255, AS-701173, AS703026, RDEA436, E6201, RO4987655, RG7167, and RG7420, or a pharmaceutically acceptable salt thereof.

Preferably, the MEK inhibitor is trametenib (N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1 (2H)-yl }phenyl)acetamide, also referred to as JPT-74057 or GSK1120212). Trametinib (GSK1120212) is described in PCT Publication No. WO05/121142The compound has been approved as Mekinist®.

According to the present disclosure, another suitable MEK inhibitor for the combination of the present disclosure is a compound 6-(4-bromo-2-fluorophenylamino)-7-fluoro-3-methyl-3H-benzoimidazole-5-carboxylic acid (2-hydroxyethoxy)-amide of formula (III)

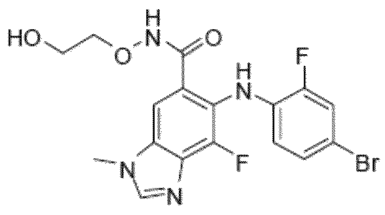

(III)

The MEK inhibitor compound 6-(4-bromo-2-fluorophenylamino)-7-fluoro-3-methyl-3H-benzoimidazole-5-carboxylic acid (2-hydroxyethoxy)-amide is described in the PCT Application No. WO 03/077914, and methods for its preparation have been described, for example, in Example 18 therein.

Additional suitable MEK inhibitor for the combination of the present disclosure is compound (S)-5-fluoro-2-(2-fluoro-4-(methylthio)phenylamino)-N-(2-hydroxypropoxy)-1-methyl-6-oxo-1,6-dihydropyridine-3-carboxamide is a compound of formula (IV)

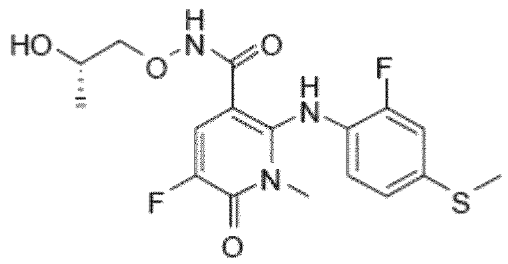

(IV)

The MEK inhibitor compound (S)-5-fluoro-2-(2-fluoro-4-(methylthio)phenylamino)-N-(2-hydroxypropoxy)-1-methyl-6-oxo-1,6-dihydropyridine-3-carboxamide is described in Example 25-BB of PCT Application No. WO2007/044084, and methods for its preparation have been described therein.

An especially preferred salt of 6-(4-bromo-2-fluorophenylamino)-7-fluoro-3-methyl-3H-benzoimidazole-5-carboxylic acid (2-hydroxyethoxy)-amide is a hydrochloride or sulfate salt. Additional pharmaceutically acceptable salts of 6-(4-bromo-2-fluorophenylamino)-7-fluoro-3-methyl-3H-benzoimidazole-5-carboxylic acid (2-hydroxyethoxy)-amide and (S)-5-fluoro-2-(2-fluoro-4-(methylthio)phenylamino)-N-(2-hydroxypropoxy)-1-methyl-6-oxo-1,6-dihydropyridine-3-carboxamide suitable for the present disclosure include the salts disclosed in PCT Application No. WO 03/077914 and PCT Application No. WO2007/044084.

Additional MEK inhibitors that may be used in the combination of the present disclosure include, but are not limited to, PD0325901 (Pfizer)(See PCT Publication No. WO02/06213), PD-184352 (Pfizer), RDEA119 (Ardea Biosciences), XI,518 (Exelexis), AS-701255 (Merck Serono), AS-701173 (Merck Serono), AS703026 (Merck Serono), RDEA436 (Ardea Biosciences, E6201 (Eisai)( See Goto et al, Journal of Pharmacology and Experimental Therapeutics, 3331(2): 485-495 (2009)), RO4987655 (Hoffmann-La Roche), RG7167, and/or RG7420.

The term "a Bcl2 inhibitor" or "a BCL2 inhibitor" or "BCL-2 inhibitor" or "Bcl-2 inhibitor" is defined herein to refer to a compound which targets, decreases or inhibits anti-apoptotic B-cell lymphoma-2 (Bcl-2) family of proteins (Bcl-2, Bcl-$X_L$, Bcl-w, Mcl-1, Bfll/A-1, and/or Bcl-B).

According to the present disclosure the pharmaceutical combination comprising the MDM2 of the invention and the Bcl2 inhibitor of the invention may further advantageously comprise a further inhibitor, which even further improves anti-tumor activity of the combination. Thus, a triple combination of MDM2 inhibitor, a MEK inhibitor and Bcl2 inhibitor caused synergistic inhibition over the drug pairs in 2/5 TP53 wild-type colorectal cancer cell models tested (Example 2, Table 5), and in four of those cell lines the triple combination showed stronger apoptosis compared to the pair wise combinations (Example 2, Figure 10).

Similarly, the pharmaceutical combinations of the present disclosure comprising the MDM2 inhibitor of the invention and the Bcl2 inhibitor of the invention may further advantageously comprise an EGFR inhibitor.

The term "an EGFR inhibitor" is defined herein to refer to a compound which targets, decreases or inhibits the activity of the epidermal growth factor family of receptor tyrosine kinases (EGFR, ErbB2, ErbB3, ErbB4 as homo- or heterodimers) or bind to EGF or EGF related ligands.

The EGFR inhibitor compound used in the combination of the present disclosure is selected from the group consisting of erlotinib, gefitinib, lapatinib, canertinib, pelitinib, neratinib, (R,E)-N-(7-chloro-1-(1-(4-(dimethylamino)but-2-enoyl)azepan-3-yl)-1H-benzo[d]imidazol-2-yl)-2-methylisonicotinamide, panitumumab, matuzumab, pertuzumab, nimotuzumab, zalutumumab, icotinib, afatinib and cetuximab, and pharmaceutically acceptable salt thereof.

Preferably, the EGFR inhibitor is erlotinib, or a pharmaceutically acceptable salt thereof.

In one embodiment, the pharmaceutical combination of the invention may further advantageously comprise the EGFR inhibitor. It has been surprisingly found that this triple combination showed stronger apoptosis compared to the pair wise combinations (Example 3, Figure 12).

In a preferred embodiment, the pharmaceutical combination comprises the MDM2 inhibitor of the invention, or a pharmaceutically acceptable salt thereof; the Bcl2 inhibitor of the invention, or a pharmaceutically acceptable salt thereof, the MEK inhibitor trametinib, or pharmaceutically acceptable salt thereof, and the EGFR inhibitor erlotinib, or a pharmaceutically acceptable salt thereof. According to the present disclosure, the pharmaceutical combinations of the invention may further advantageously comprise a PI3K inhibitor.

The term "a phosphatidylinositol 3-kinase inhibitor" or "a PI3K inhibitor" is defined herein to refer to a compound which targets, decreases or inhibits PI3-kinase. PI3-kinase activity has been shown to increase in response to a number of hormonal and growth factor stimuli, including insulin, platelet-derived growth factor, insulin-like growth factor, epidermal growth factor, colony-stimulating factor, and hepatocyte growth factor, and has been implicated in processes related to cellular growth and transformation.

Phosphatidylinositol -3-kinase (PI3K) inhibitors suitable for the present disclosure are selected from the group consisting of 2-methyl-2-[4-(3-methyl-2-oxo-8-quinolin-3-yl-2,3-dihydro-imidazo[4,5-c]quinolin-1-yl)-phenyl]-propionitrile, or a pharmaceutically acceptable salt thereof, 5-(2,6-di-morpholin-4-yl-pyrimidin-4-yl)-4-trifluoromethyl-pyridin-2-ylamine, or a pharmaceutically acceptable salt thereof; and (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), or a pharmaceutically acceptable salt thereof.

WO2006/122806 describes imidazoquinoline derivatives, which have been described to inhibit the activity of PI3K. The compound 2-methyl-2-[4-(3-methyl-2-oxo-8-quinolin-3-yl-2,3-dihydro-imidazo[4,5-c]quinolin-1-yl)-phenyl]-propionitrile has the chemical structure of formula (V)

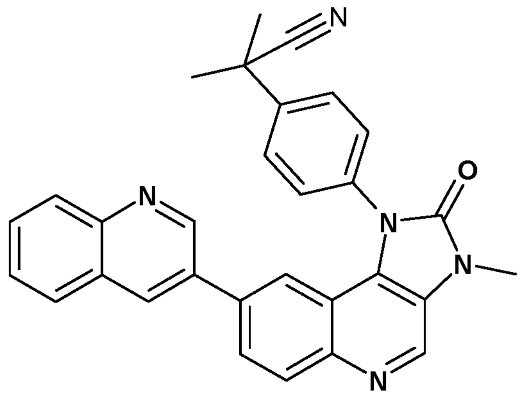

(V).

The compound, its utility as a PI3K inhibitor and synthesis of 2-methyl-2-[4-(3-methyl-2-oxo-8-quinolin-3-yl-2,3-dihydro-imidazo[4,5-c]quinolin-1-yl)-phenyl]-propionitrile and its monotosylate salt are described in WO2006/122806for instance in Example 7 and Example 152-3 respectively. The compound 2-methyl-2-[4-(3-methyl-2-oxo-8-quinolin-3-yl-2,3-dihydro-imidazo[4,5-c]quinolin-1-yl)-phenyl]-propionitrile may be present in the form of the free base or any pharmaceutically acceptable salt thereto. Preferably, 2-methyl-2-[4-(3-methyl-2-oxo-8-quinolin-3-yl-2,3-dihydro-imidazo[4,5-c]quinolin-1-yl)-phenyl]-propionitrile is in the form of its monotosylate salt.

WO07/084786 describes specific pyrimidine derivatives which have been found to inhibit the activity of PI3K. The compound 5-(2,6-di-morpholin-4-yl-pyrimidin-4-yl)-4-trifluoromethyl-pyridin-2-ylamine has the chemical structure of formula (VI)

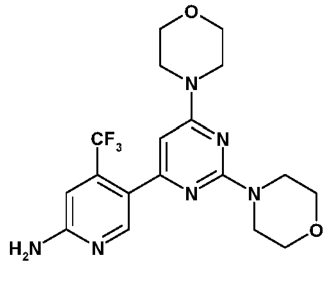

(VI).

The compound, its salts, its utility as a PI3K inhibitor and synthesis of the compound 5-(2,6-di-morpholin-4-yl-pyrimidin-4-yl)-4-trifluoromethyl-pyridin-2-ylamine are described in WO 2007/084786, for instance in Example 10. The compound 5-(2,6-di-morpholin-4-yl-pyrimidin-4-yl)-4-trifluoromethyl-pyridin-2-ylamine may be present in the form of the free base or any pharmaceutically acceptable salt thereto. Preferably, 5-(2,6-di-morpholin-4-yl-pyrimidin-4-yl)-4-trifluoromethyl-pyridin-2-ylamine is in the form of its hydrochloride salt.

WO2010/029082 describes specific 2-carboxamide cycloamino urea derivatives which have been found to be highly selective for the alpha isoform of PI3K and can be added to the combinations of the present disclosure. The compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) has the chemical structure of formula (VII)

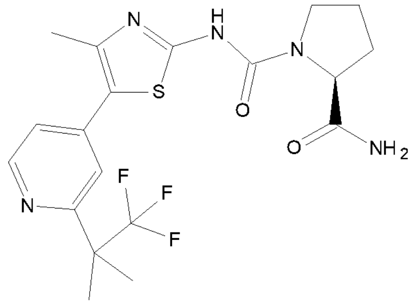

(VII).

The compound, its salts, its utility as an alpha-isoform selective PI3K inhibitor and synthesis of the compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) are described in WO2010/029082, for instance in Example 15. The compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) may be present in the form of the free base or any pharmaceutically acceptable salt thereto. Preferably, (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide)is in the form of its free base.

Preferably, the PI3K inhibitor compound used in the combination of the present disclosure is (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), or any pharmaceutically acceptable salt thereof.

In one embodiment, the pharmaceutical combination comprising the MDM2 inhibitor and the Bcl2 inhibitor may further advantageously comprise the PI3K inhibitor. It has been surprisingly found that this triple combination synergistic inhibition (over the drug pairs in 2/5 cell models tested (Example 4, Table 9) and showed stronger apoptosis compared to the pair wise combinations (Example 4, Figure 14).

Furthermore, according to the present disclosure, the pharmaceutical combinations of the invention may further advantageously comprise a BRAF inhibitor.

Furthermore, the pharmaceutical combination of the present invention may advantageously comprise (a) the MDM2 inhibitor of the invention, (b) a MEK inhibitor, (c) the Bcl2 inhibitor of the invention, and (d) a BRAF inhibitor.

The term "a BRAF inhibitor" is defined herein to refer to a compound which targets, decreases or inhibits the activity of serine/threonine-protein kinase B-Raf.

The pharmaceutical combination according to any one of the preceding claims, wherein the BRAF inhibitor is selected from the group consisting of RAF265, dabrafenib (S)-methyl-1-(4-(3-(5-chloro-2-fluoro-3-(methylsulfonamido)phenyl)-1-isopropyl-1H-pyrazol-4-yl)pyrimidin-2-ylamino)propan-2-ylcarbamate, methyl N-[(2S)-1-({4-[3-(5-chloro-2-fluoro-3-methanesulfonamidophenyl)-1-(propan-2-yl)-1H-pyrazol-4-yl]pyrimidin-2-yl}amino)propan-2-yl]carbamate and vemurafenib, or a pharmaceutically acceptable salt thereof.

According to the present disclosure, the BRAF inhibitor is preferably dabrafenib, or a pharmaceutically acceptable salt thereof. In one embodiment, the BRAF inhibitor added to the combination is RAF265.

The combination of the present disclosure can further comprise a CDK4/6 inhibitor. "Cyclin dependent kinase 4/6 (CDK4/6) inhibitor" as defined herein refers to a small molecule that interacts with a cyclin-CDK complex to block kinase activity. The Cyclin-dependent kinases (CDK) is a large family of protein kinases that regulate initiation, progression, and completion of the mammalian cell cycle. Preferably, the CDK4/6 inhibitor is 7-cyclopentyl-N,N-dimethyl-2-((5-(piperazin-1-yl)pyridin-2-yl)amino)-7H-pyrrolo[2,3-d]pyrimidine-6-carboxamide, or pharmaceutically acceptable salt thereof.

The term "pharmaceutically acceptable salts" refers to salts that retain the biological effectiveness and properties of the compound and which typically are not biologically or otherwise undesirable. The compound may be capable of

forming acid addition salts by virtue of the presence of an amino group.

Unless otherwise specified, or clearly indicated by the text, reference to therapeutic agents useful in the pharmaceutical combination of the present disclosure includes both the free base of the compounds, and all pharmaceutically acceptable salts of the compounds.

The term "combination" or "pharmaceutical combination" is defined herein to refer to either a fixed combination in one dosage unit form, a non-fixed combination or a kit of parts for the combined administration where the therapeutic agents may be administered together, independently at the same time or separately within time intervals, which preferably allows that the combination partners show a cooperative, e.g. synergistic effect. Thus, the single compounds of the pharmaceutical combination of the present disclosure could be administered simultaneously or sequentially.

Furthermore, the pharmaceutical combination of the present disclosure may be in the form of a fixed combination or in the form of a non-fixed combination.

The term "fixed combination" means that the therapeutic agents, e.g., the single compounds of the combination, are in the form of a single entity or dosage form.

The term "non-fixed combination" means that the therapeutic agents, e.g., the single compounds of the combination, are administered to a patient as separate entities or dosage forms either simultaneously or sequentially with no specific time limits, wherein preferably such administration provides therapeutically effective levels of the two therapeutic agents in the body of the subject, e.g., a mammal or human in need thereof.

The pharmaceutical combinations can further comprise at least one pharmaceutically acceptable carrier. Thus, the present disclosure relates to a pharmaceutical composition comprising the pharmaceutical combination of the present disclosure and at least one pharmaceutically acceptable carrier.

As used herein, the term "carrier" or "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Generally, the term "pharmaceutical composition" is defined herein to refer to a mixture or solution containing at least one therapeutic agent to be administered to a subject, e.g., a mammal or human. The present pharmaceutical combinations can be formulated in a suitable pharmaceutical composition for enteral or parenteral administration are, for example, those in unit dosage forms, such as sugar-coated tablets, tablets, capsules or suppositories, or ampoules. If not indicated otherwise, these are prepared in a manner known per se, for example by means of various conventional mixing, comminution, direct compression, granulating, sugar-coating, dissolving, lyophilizing processes, or fabrication techniques readily apparent to those skilled in the art. It will be appreciated that the unit content of a combination partner contained in an individual dose of each dosage form need not in itself constitute an effective amount since the necessary effective amount may be reached by administration of a plurality of dosage units. The pharmaceutical composition may contain, from 0.1 % to 99.9%, preferably from 1 % to 60 %, of the therapeutic agent(s). One of ordinary skill in the art may select one or more of the aforementioned carriers with respect to the particular desired properties of the dosage form by routine experimentation and without any undue burden. The amount of each carriers used may vary within ranges conventional in the art. The following references disclose techniques and excipients used to formulate oral dosage forms. See The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2003). These optional additional conventional carriers may be incorporated into the oral dosage form either by incorporating the one or more conventional carriers into the initial mixture before or during granulation or by combining the one or more conventional carriers with granules comprising the combination of agents or individual agents of the combination of agents in the oral dosage form. In the latter embodiment, the combined mixture may be further blended, e.g., through a V-blender, and subsequently compressed or molded into a tablet, for example a monolithic tablet, encapsulated by a capsule, or filled into a sachet. Clearly, the pharmaceutical combinations of the present disclosure can be used to manufacture a medicine.

The present disclosure relates to such pharmaceutical combinations or pharmaceutical compositions that are particularly useful as a medicine.

Specifically, the combinations or compositions of the present disclosure can be applied in the treatment of cancer.

The term "treatment" as used herein comprises a treatment relieving, reducing or alleviating at least one symptom in a subject, increasing progression-free survival, overall survival, extending duration of response or delaying progression of a disease. For example, treatment can be the diminishment of one or several symptoms of a disorder or complete eradication of a disorder, such as cancer. Within the meaning of the present disclosure, the term "treatment" also denotes to arrest, delay the onset (i.e., the period prior to clinical manifestation of a disease) and/or reduce the risk of developing or worsening a disease in a patient, e.g., a mammal, particularly the patient is a human. The term "treatment" as used herein comprises an inhibition of the growth of a tumor incorporating a direct inhibition of a primary tumor growth and / or the systemic inhibition of metastatic cancer cells.

A "subject," "individual" or "patient" is used interchangeably herein, which refers to a vertebrate, preferably a mammal, more preferably a human. Mammals include, but are not limited to, mice, simians, humans, farm animals, sport animals, and pets.

The term "a therapeutically effective amount" of a compound (e.g. chemical entity or biologic agent) of the present disclosure refers to an amount of the compound of the present disclosure that will elicit the biological or medical response of a subject, for example, reduction or inhibition of an enzyme or a protein activity, or ameliorate symptoms, alleviate conditions, slow or delay disease progression, or prevent a disease, etc. In one embodiment a therapeutically effective amount in vivo may range depending on the route of administration, between 0.1-500 mg/kg, or between 1-100 mg/kg.

The optimal dosage of each combination partner for treatment of a cancer can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art. The amount of each combination partner that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone.

Frequency of dosage may vary depending on the compound used and the particular condition to be treated or prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

A therapeutic amount or a dose of (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one may range between 100 and 1500 mg every three weeks, particularly between 100 and 800 mg every three weeks, or between 50 and 600 mg daily, when administered per os. A therapeutic amount or a dose of (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one can be 400 mg, more preferably is 300 mg for daily administration for the first 21 days of every 28 day cycle. Alternatively, a total therapeutic amount or a total dose of (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one is 560 mg per cycle (40 mg qd 2 wks on / 2 wks off, or 80 mg qd 1 wk on / 3 wks off). Intravenous doses would need to be lowered accordingly.

The recommended dose of the MEK inhibitor trametinib is 2 mg daily. The management of adverse reactions may require dose reduction up to 1 mg daily.

The MEK inhibitor compound 6-(4-bromo-2-fluorophenylamino)-7-fluoro-3-methyl-3H-benzoimidazole-5-carboxylic acid (2-hydroxyethoxy)-amide may be administered to a suitable subject daily in single or divided doses at an effective dosage in the range of 0.001 to 100 mg per kg body weight per day, preferably 1 to 35 mg/kg/day, in single or divided doses. For a 70 kg human, this would amount to a preferable dosage range of 0.05 to 7 g/day, preferably 0.05 to 2.5 g/day.

The MEK inhibitor compound (S)-5-fluoro-2-(2-fluoro-4-(methylthio)phenylamino)-N-(2-hydroxypropoxy)-1-methyl-6-oxo-1,6-dihydropyridine-3-carboxamide may be administered daily to a suitable subject in single or divided doses at an effective dosage in the range of 0.001 to 100 mg per kg body weight per day, preferably 1 mg/kg/day to 35 mg/kg/day, in single or divided doses. For a 70 kg human, this would amount to a preferable dosage range of 0.07 to 2.45 g/day, preferably 0.05 to 1.0 g/day.

An effective dose of the Bcl-2 inhibitor navitoclax may range from 100 mg to 500 mg daily. The dose may be reduced or a 150 mg 7-day lead-in dose employed. After the lead-in dose a 325 mg dose or up to 425 mg dose can be administered daily.

The recommended dose of the EGFR inhibitor erlotinib is 100 mg or 150 mg daily.

The PI3K inhibitor compound (S)-pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) is generally administered orally at a dose in the range from from 30 mg to 450 mg per day, for example 100 to 400 mg per day in a human adult. The daily dose can be administered on a qd or bid schedule. (S)-pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) may administered to a suitable subject daily in single or divided doses at an effective

dosage in the range of 0.05 to 50 mg per kg body weight per day, preferably 0.1-25 mg/kg/day, more preferably from 0.5-10 mg/kg/day , in single or divided doses. For a 70 kg human, this would amount to a preferable dosage range of 35-700 mg per day. More preferably, the dosage range is of 35 - 400 mg per day.

The PI3K inhibitor compound 2-methyl-2-[4-(3-methyl-2-oxo-8-quinolin-3-yl-2,3-dihydro-imidazo[4,5-c]quinolin-1-yl)-phenyl]-propionitrile is generally administered orally at a dose in the range from 100 mg to 1200 mg, or 200 mg to 1000 mg, or 300 mg to 800 mg, or 400 mg to 600 mg per day in a human adult. The daily dose can be administered on a qd or bid schedule.

The PI3K inhibitor compound 5-(2,6-di-morpholin-4-yl-pyrimidin-4-yl)-4-trifluoromethyl-pyridin-2-ylamine is generally administered orally at a dose in the range from 30 mg to 300 mg, or 60 mg to 120 mg, or 100 mg per day in a human adult. The daily dose can be administered on a qd or bid schedule.

The recommended dose of the BRAF inhibitor dabrafenib is 150 mg orally twice daily as a single agent or in combination with trametinib 2 mg orally once daily.

It is understood that each therapeutic agent may be conveniently administered, for example, in one individual dosage unit or divided into multiple dosage units. It is further understood that that each therapeutic agent may be conveniently administered in doses once daily or doses up to four times a day.

The term "cancer" is used herein to mean a broad spectrum of tumors, in particular solid tumors. Examples of such tumors include, but are not limited to a benign or malignant tumor of the lung (including small cell lung cancer and non-small-cell lung cancer), bronchus, prostate, breast (including sporadic breast cancers and sufferers of Cowden disease), pancreas, gastrointestinal tract, colon, rectum, colon carcinoma, colorectal cancer, thyroid, liver, biliary tract, intrahepatic bile duct, hepatocellular, adrenal gland, stomach, gastric, glioma, glioblastoma, endometrial, kidney, renal pelvis, bladder, uterus, cervix, vagina, ovary, multiple myeloma, esophagus, neck or head, brain, oral cavity and pharynx, larynx, small intestine, a melanoma, villous colon adenoma, a sarcoma, a neoplasia, a neoplasia of epithelial character, a mammary carcinoma, basal cell carcinoma, squamous cell carcinoma, actinic keratosis, polycythemia vera, essential thrombocythemia, a leukemia (including acute myelogenous leukemia, chronic myelogenous leukemia, lymphocytic leukemia, and myeloid leukemia), a lymphoma (including non-Hodgkin lymphoma and Hodgkin's lymphoma), myelofibrosis with myeloid metaplasia, Waldenstroem disease, and Barret's adenocarcinoma.

Preferably, the cancer is colorectal cancer, melanoma, liposarcoma, glioblastoma, neuroblastoma, lymphoma or leukemia. In a preferred embodiment the cancer is colorectal cancer. The term "colorectal cancer", as used herein, refers to cancer in the colon or rectum, also known as colon cancer, rectal cancer or bowel cancer. In one embodiment, the present disclosure relates to metastatic colorectal cancer.

The combination is expected to achieve superior effects in functional p53 or p53 wild-type cancers. The TP53 gene is one of the most frequently mutated genes in human cancers. Thus, tumor suppressor p53 is functionally impaired by mutation or deletion in nearly 50% of human cancers. In the remaining human cancers, p53 retains wild-type status but its function is inhibited by its primary cellular inhibitor, the murine double minute 2 (Mdm2, MDM2; HDM2 (human homolog of murine double minute 2)). Mdm2 is a negative regulator of the p53 tumor suppressor. Mdm2 protein functions both as an E3 ubiquitin ligase, that leads to proteasomal degradation of p53, and an inhibitor of p53 transcriptional activation. Often Mdm2 is found amplified in p53 wild-type tumors. Because the interaction between Mdm2 and p53 is a primary mechanism for inhibition of the p53 function in cancers, which are retaining wild-type p53, the combination of the present disclosure comprising the MDM2 inhibitor is particularly useful for treatment of functional p53 or p53 wild-type cancers.

In addition, the efficacy of the combination is expected to be increased in cancer, which is characterized by one or more of KRAS mutation and/or BRAF mutation and/or MEK1 mutation and/or PIK3CA mutation and/or PIK3CA overexpression.

Patients with colorectal cancer harboring KRAS or BRAF mutations, which together make up 50%-60% of reported colorectal cancer cases (Fearon 2011), are generally associated with a poor prognosis (Arrington, Heinrich et al. 2012, Safaee Ardekani, Jafarnejad et al. 2012). The combinations of this disclosure are particularly useful for treatment of cancer, which comprises one or more of KRAS mutation or one or more of BRAF mutation.

Examples of BRAF mutations include, but not limited to V600E, R461I, I462S, G463E, G463V, G465A, G465E, G465V, G468A, G468E, N580S, E585K, D593V, F594L, G595R, L596V, T598I, V599D, V599E, V599K, V599R, V600K, A727V. Most of these mutations are clustered to two regions: the glycine-rich P loop of the N lobe and the activation segment and flanking regions. V600E mutation has been detected in a variety of cancers, and is due to a substitution of thymine with adenine at nucleotide 1799. This leads to valine (V) being substituted for by glutamate (E) at codon 600 (now referred to as V600E).

MEK1 mutation may be, for example, MEK1 S72G mutation.

Examples of PIK3CA mutation and/or PIK3CA overexpression include, but not limited to , amplification of the alpha isoform of PI3K, somatic mutation of PIK3CA, germline mutations or somatic mutations of PTEN, mutations and translocation of p85$\alpha$ that serve to up-regulate the p85-p110 complex, or amplification or overexpression of the beta isoform of PI3K.

The pharmaceutical combination of the present disclosure is particularly useful for the treatment of a cancer, particularly colorectal cancer, wherein the cancer is resistant to a treatment with an EGFR inhibitor, or is developing a resistance to a treatment with an EGFR inhibitor, or is under high risk of developing a resistance to a treatment with an EGFR inhibitor, particularly wherein the EGFR inhibitor is selected from the group consisting of erlotinib, gefitinib and afatinib.

The pharmaceutical combination of the present disclosure is also suitable for the treatment of poor prognosis patients, especially such poor prognosis patients having a cancer, particularly colorectal cancer, which becomes resistant to treatment employing an EGFR inhibitor, e.g. a cancer of such patients who initially had responded to treatment with an EGFR inhibitor and then relapsed. In a further example, said patient has not received treatment employing a FGFR inhibitor. This cancer may have acquired resistance during prior treatment with one or more EGFR inhibitors. For example, the EGFR targeted therapy may comprise treatment with gefitinib, erlotinib, lapatinib, XL-647, HKI-272 (Neratinib), BIBW2992 (Afatinib), EKB-569 (Pelitinib), AV-412, canertinib, PF00299804, BMS 690514, HM781-36b, WZ4002, AP-26113, cetuximab, panitumumab, matuzumab, trastuzumab, pertuzumab, or a pharmaceutically acceptable salt thereof. In particular, the EGFR targeted therapy may comprise treatment with gefitinib, erlotinib, and afatinib. The mechanisms of acquired resistance include, but are not limited to, developing a second mutation in the EGFR gene itself, e.g. T790M, EGFR amplification; and / or FGFR deregulation, FGFR mutation, FGFR ligand mutation, FGFR amplification, or FGFR ligand amplification.

The following Examples illustrates the disclosure described above, but is not, however, intended to limit the scope of the disclosure in any way. Other test models known as such to the person skilled in the pertinent art can also determine the beneficial effects of the claimed disclosure.

## Examples

"COMPOUND A", "COMPOUND B" or the like denote herein specific compounds. Denotation of a respective compound may not be the same for all examples or combinations. Rather, the compounds are denoted in each example anew.

**Reference Example 1: The in vitro effect on proliferation of combining a MDM2 inhibitor and a MEK inhibitor.**

This study was designed to explore an in vitro effect on proliferation of combining the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A) or the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one (COMPOUND B) with the MEK inhibitor trametinib (COMPOUND C) in TP53 wild-type colorectal cancer cell lines.

### *METHODS*

COMPOUNDS A, B, and C were dissolved in 100% DMSO (Sigma, Catalog number D2650) at concentrations of 20mM and stored at -20oC until use.

Colorectal cancer cell lines used for this study were obtained, cultured and processed from the commercial vendors ATCC, ECACC, DSMZ, and CellBank Australia (Table 1). All cell line media were supplemented with 10% FBS (HyClone, Catalog number SH30071.03). Media for LIM2405 was additionally supplemented with 0.6ug/ml Insulin (SIGMA, Catalog number 19278), 1ug/ml Hydrocortisone (SIGMA, Catalog number H0135), and 10μM 1-Thioglycerol (SIGMA, Catalog number M6145).

| Cell line | Driver mutations | Source | Source Cat Num | Medium | Medium Vendor | Medium Cat Num | #Cells | Treatment [h] |
|---|---|---|---|---|---|---|---|---|
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| COLO-678 | KRAS | DSMZ | ACC-194 | RPMI | ATCC | 30-2001 | 1250 | 96 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |
| LIM2405 | BRAF | CellBank Australia | CBA-0165 | RPMI | ATCC | 30-2001 | 750 | 72 |
| SW48 | MEK1 | ATCC | CCL-231 | RPMI | ThermoFisher | 22400-071 | 1250 | 96 |

**Table 1.** Cell line information.

Cell lines were cultured in 37°C and 5% $CO_2$ incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

To test the effect of the combination of COMPOUND A or COMPOUND B with COMPOUND C on cell proliferation cells were plated in black 384-well microplates with clear bottom (Matrix/Thermo Scientific, Catalog number 4332) in 50ul media per well at cell densities between 500 and 1250 cells/well (Table 1) and allowed to incubate at 37 degrees, 5% $CO_2$ for 24h. After 24h one 384-well plate per cell line was prepared for cell counting by microscopy (see below) without receiving treatment (= 'baseline'). The other cell plates were treated using a HP D300 Digital Dispenser (Tecan) generating 6-point dose response curves with 2.5X dilution steps.

COMPOUND A was used over a final concentration range of 51nM - 5uM, COMPOUND B over a final concentration range of 10nM - 1uM, and COMPOUND C over a final concentration range of 1nM - 100nM.

For the combinations of COMPOUND A or COMPOUND B with COMPOUND C the single agents were combined at 6 single agent doses generating a dose matrix of 6x6 = 36 combination treatments. Additionally, negative controls (DMSO = 'vehicle') and positive controls (Staurosporine = killing cells, 7-point 1:2 dilution series for a dose range of 16nm - 1uM) were transferred as treatment controls. Cells were treated for 72h to 96h depending on their doubling time (Table 1). At the end of the treatment cells were prepared for cell counting by microscopy. Cells were fixed and permeabilised for 45 minutes in 4% PFA (Electron Microscopy Sciences, Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 $\mu$g/ml. Cells were washed three times with PBS and then plates were heat-sealed using a PlateLoc (Agilent Technologies) with aluminum seals (Agilent Technologies, Catalog number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

To test the effects of the combinations on the induction of apoptosis a Caspase 3/7 assay was performed using a similar experimental setup as for the proliferation assay described above and just testing the combination of COMPOUND A with COMPOUND C. Compounds were arrayed in drug master plates (Greiner, Catalog number 788876) and serially diluted 3-fold (7 steps) at 2000X concentration. Cells were treated by transferring 25nl of the 2000X compound from drug master plates using an ATS acoustic liquid dispenser (ECD Biosystems) to 50uL cells, resulting in a final 1X concentration.

COMPOUND A was used over a final concentration range of 13nM - 10uM, and COMPOUND C over a final concentration range of 0.4nM - 0.3uM. Additionally, negative controls (DMSO = 'vehicle') and positive controls (Staurosporine = killing cells, 7-point 1:2 dilution series for a dose range of 16nm - 1uM) were transferred as treatment controls.

After compound addition 50nl of 2mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 1), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy and images as described above for the cell proliferation assay.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figures 1 and 2 denoted as 'Normalized cell count' on the y-axis. Synergy of the combinations was assessed by isobologram analysis (Greco, Bravo et al. 1995)(Figure 3) and by calculation of combination indices (Chou, Talalay 1984)(Table 2), which tests for synergy under the Loewe model (Loewe 1928). The CI analysis was done for a 75% iso-effect level (75% inhibition under single agent treatments compared to the combination treatment). The 'best CI' (red points in Figure 3 and Table2) is the lowest combination index observed for this combination

in a particular cell line.

**The combinations index (CI) is an indicator for the combination effect with**

CI < 1: synergy
CI = 1: additive effect
CI > 1: antagonism,

e.g. a combination index of 0.5 indicates that in combination only half of each single agent is required when compared to the required single agent doses alone to reach the same effect (here 75% inhibition).

IC75 is the compound concentration that results in 75% of the cell counts relative to DMSO. IC75 calculations (see Table 2) were done by performing a 3-parameter logistic regression on the data.

| Cell | IC75 COMPOUND A | IC75 COMPOUND B | IC75 trametinib (C) | COMBINATION A+C best CI | COMBINATION B+C best CI |
|---|---|---|---|---|---|
| colo678 | 3.13 | >1 | >0.1 | 0.18 | 0.19 |
| rko | 1.75 | 0.54 | >0.1 | 0.26 | 0.21 |
| gp2d | 1.11 | 0.23 | >0.1 | 0.30 | 0.24 |
| lovo | 1.32 | 0.34 | 0.018 | 0.35 | 0.31 |
| lim2405 | 0.81 | 0.20 | 0.008 | 0.47 | 0.34 |
| ls180 | 0.88 | 0.20 | 0.014 | 0.60 | 0.55 |
| hct116 | 0.59 | 0.12 | 0.029 | 0.61 | 0.48 |
| sw48 | 1.01 | 0.26 | 0.003 | 0.65 | 0.63 |

**Table 2.** Single agent IC75 values for each compound and best combination indices (best CI) for the combinations of COMPOUND A and trametinib and COMPOUND B and trametinib.

The compound's effect on apoptosis was determined by calculating the percentage of cells with activated Caspase 3/7 per treatment and time point relative to the raw cell counts (before subtraction of debris) (y-axis in Figure 4). Cell counts at time points that were not experimentally measured were obtained by regression analysis by fitting a linear model for log-transformed cell counts at day 0 and the end of the treatment (assuming exponential cell growth).

For colony formation assays (Figure 5) cells were plated in 1 ml medium in 12-well tissue culture-treated plates (Costar, Catalog number 3513): for COLO-678 6000 cells/well, SW48 5000 cells/well, GP2d 2000 cells/well, LoVo 2500 cells/well, LS-180 2500 cells/well, LIM2405 2500 cells/well, RKO 1000 cells/well, and for HCT-116 1000 cells/well. Cells were grown for 72h before addition of compounds, and treatments were refreshed every 48h (in fresh medium) for up to 14 days using a HP D300 Digital Dispenser (Tecan). At the end of the treatment cells were washed in PBS once, fixed and stained for 30 minutes at room temperature using a solution containing 4% PFA (Electron Microscopy Sciences, Catalog number 15714) and 2 mg/ml Crystal Violet (EMD, Catalog number 192-12), and washed 3 times with water. Plates were dried overnight and the scanned using an Odyssee imager (Licor). ImageStudio software (Licor) was used to quantify the crystal violet signal for Figure 5. For significance test see Table 3.

| Cell | COMPOUND A + trametinib | COMPOUND A | trametinib |
|---|---|---|---|
| COLO-678 | Combination (LL) | *** | *** |
| COLO-678 | Combination (LH) | *** | *** |
| COLO-678 | Combination (HL) | *** | *** |
| COLO-678 | Combination (HH) | *** | *** |
| RKO | Combination (LL) | *** | ** |
| RKO | Combination (LH) | *** | *** |
| RKO | Combination (HL) | *** | *** |
| RKO | Combination (HH) | *** | *** |
| GP2d | Combination (LL) | *** | *** |
| GP2d | Combination (LH) | *** | *** |
| GP2d | Combination (HL) | ** | *** |
| GP2d | Combination (HH) | ** | *** |
| LoVo | Combination (LL) | *** | *** |
| LoVo | Combination (LH) | *** | ** |
| LoVo | Combination (HL) | *** | *** |
| LoVo | Combination (HH) | *** | ** |
| LIM2405 | Combination (LL) | *** | *** |
| LIM2405 | Combination (LH) | *** | *** |
| LIM2405 | Combination (HL) | * | *** |
| LIM2405 | Combination (HH) | ** | *** |
| LS-180 | Combination (LL) | *** | *** |
| LS-180 | Combination (LH) | *** | ** |
| LS-180 | Combination (HL) | ** | *** |
| LS-180 | Combination (HH) | *** | ** |
| HCT-116 | Combination (LL) | *** | *** |
| HCT-116 | Combination (LH) | *** | *** |
| HCT-116 | Combination (HL) | *** | *** |
| HCT-116 | Combination (HH) | *** | *** |
| SW48 | Combination (LL) | *** | *** |
| SW48 | Combination (LH) | *** | ** |
| SW48 | Combination (HL) | *** | *** |
| SW48 | Combination (HH) | *** | *** |

**Table 3.** Significance of difference of colony formation assay results (Figure 4) of COMPOUND A and trametinib combination when compared to the corresponding doses of COMPOUND A or trametinib alone (one-tailed t-test). *$p<0.05$, **$p<0.01$, ***$p<0.001$.

For cell cycle analysis (Figure 6) cells were plated in 10ml medium in 10cm tissue culture-treated dishes (Corning, Catalog number 430167): for COLO-678 3.5 million cells, SW48 2.75 million cells, GP2d 2.5 million cells, LoVo 2.5 million cells, LS-180 2.5 million cells, LIM2405 1.5 million cells, RKO 1.5 million cells, and for HCT-116 2 million cells. Drug treatments were carried out manually after 24h. Samples were collected 24h after treatment by collecting the supernatant and harvesting the cells by trypsinization using 0.25% Trypsin-EDTA (GIBCO, Catalog number 25200). Cells were pelleted, washed once with PBS, and pelleted again before fixation in 1 ml ice-cold 70% ethanol (added drop

wise to the pellet while vortexing) for 30 minutes at 4°C. Next, cells were pelleted at higher speed (850xg) for 5 min and the pellet was washed twice in phosphate-citrate buffer (0.2M NA2HPO4, 0.1M citric acid, adjusted to pH 7.8) and centrifuged at the same speed (for 5 minutes). The pellet was finally dissolved in 0.5 ml of PI/RNase staining buffer (BD Pharmingen, Catalog number 550825). After 15 minutes incubation at RT the cell cycle was analyzed on a BD FACSCanto II system (analyzing 10,000 events per condition). Data was analyzed using the FlowJo software.

For Western blots (Figure 7) cells were plated in 10ml medium in 10cm tissue culture-treated dishes (Corning, Catalog number 430167): for COLO-678 8 million cells, SW48 4 million cells, GP2d 3 million cells, LoVo 4 million cells, LS-180 4.5 million cells, LIM2405 2 million cells, RKO 3.5 million cells, and for HCT-116 3.5 million cells. Cells were grown for 24h before addition of compounds. After 24h treatment cells were collected in ice-cold PBS by scraping, lysed in lysis buffer (Cell Signaling, Catalog number 9803) containing phosphatase inhibitors (Roche, Catalog number 04906837001) and protease inhibitors (Roche, Catalog number 04693116001) for 30 minutes. Lysates were quantified using the BCA protein assay kit (ThermoFisher, Catalog number 23225), the concentration normalized, loading buffer added (ThermoFisher, NP0007), 25-50ug of protein loaded on precasted 4-15% polyacrylamide gradient gels (Biorad, Catalog number 5671084), and run on an electrophoresis system (Biorad) for 30-35min at 300V. The protein was transferred on nitrocellulose membranes using the iBlot transfer system (Invitrogen) and the iBlotGel transfer kit (ThermoFisher, Catalog number IB301001). Proteins shown in Figure 5 were detected using the following primary antibodies: p53 (Santa Cruz Biotechnology, sc-126, 1:500), MDM2 (CalBiochem, #OP46, 1:500), ERK (Cell Signaling Technology, #4695, 1:1000), pERK (Cell Signaling Technology #4370, 1:1000), p21 (Cell Signaling Technology, #2947, 1:1000), p27 (Santa Cruz Biotechnology, sc-528, 1:500), CyclinD1 (Santa Cruz Biotechnology, sc-718, 1:500), BIM (Cell Signaling Technology, #2819, 1:500), cPARP (Cell Signaling Technology, #9541, 1:500), PUMA (Cell Signaling Technology, #4976, 1:500), and beta-actin (Ambion, AM4302, 1:10000). The following secondary antibodies were used: HRP goat anti rabbit (Biorad, 170-5046, 1:10000), HRP goat anti mouse (Biorad, 170-5047, 1:10000), and IRDye® 800CW Goat anti-Mouse (Licor, 925-32210, 1:10000). Membranes were developed on film (Carestream, Catalog number 178 8207) on a developer (Kodak X-OMAT 2000A) or (for beta-actin) imaged on an Odyssee imager (Licor).

For qRT-PCR analysis (Figure 8) cells were plated in 6-well tissue culture-treated plates (Corning, Catalog number 3516): for COLO-678 0.75 million cells, SW48 0.5 million cells, GP2d 0.4 million cells, LoVo 0.4 million cells, LS-180 0.4 million cells, LIM2405 0.25 million cells, RKO 0.25 million cells, and for HCT-116 0.3 million cells. Drug treatments were carried out manually after 24h. Samples were collected 10h after treatment. The supernatant was removed, cells were washed once with PBS, and then the RNeasy Mini Kit (Qiagen, Catalog number 74104) was used for RNA extraction. RNA was quantified using a NanoDrop 1000 and 1 μg total RNA was used for cDNA synthesis using the iScript cDNA synthesis kit (Biorad, Catalog number 170-8891). Taqman qRT-PCR was performed in 384 well plates on an ABI ViiA 7 (Applied Biosystems). Per reaction 6 μl of a 2x PCR master mix (ThermoFisher, Catalog number 435 2042), 0.6 μl beta-actin primer/probe set (20X), 0.6 μl target primer/probe set (20X), and 4.8 μl cDNA (1:4 dilution of product from cDNA synthesis). The following program was run: 2 minutes at 50°C, 10 min at 95°C, and the 40 cycles with 10 seconds at 95°C and 1 minute at 60°C. The following probe/primer sets (ThermoFisher TaqMan gene expression assays) were used: CDKN1A/p21 (Hs00355782_m1), BAX (Hs00180269_m1), BBC3/PUMA (Hs00248075_m1), BMF (Hs00372937_m1), NOXA1 (Hs00736699_m1).

## ***RESULTS***

In this report the efficacies of two MDM2 inhibitors (COMPOUND A and COMPOUND B) and the MEK inhibitor trametinib (COMPOUND C) were assessed individually and in combination in a total of 8 TP53 wild type colorectal cancer cell lines. Five of the lines were KRAS mutant (GP2d, LS-180, HCT-116, LoVo, COLO-678), two lines were BRAF mutant (RKO, LIM2405), and one line mutant in MEK1 (SW48). Four of the lines were also mutant for PIK3CA (GP2d, RKO, LS-180, HCT-116) (Table 1). COMPOUND A as single agent inhibited the growth of all cell lines with sub-micromolar to micromolar IC75 values, COMPOUND B with sub-micromolar IC75 values (except for COLO-678), and COMPOUND C with nanomolar IC75 values (except for COLO-678, RKO, and GP2d) (Figures 1 and 2, Table 2). The combination treatment caused synergistic inhibition (according to the Loewe model) in all cell models tested as indicated by the combination indices (CI) (Figure 3 and Table 2). Further mechanistic studies focused on the combination of COMPOUND A with trametinib. The combination showed weak induction of apoptosis (assessed by measuring Caspase 3/7 induction) (Figure 4). The combination prevented the outgrowth of clones in colony formation assays significantly better than each of the single agents, also showing the long-term efficacy of the combination (Figure 5 and Table 3). FACS analysis was performed after 24h treatment and showed that MDM2 inhibition depleted cells in the S-phase and arrested them in G1 and/or G2 phases of the cell cycle (Figure 6). The responses to MEK inhibition were more cell line dependent, but in the majority of models it resulted in increased G1 populations. The combination mainly showed S-phase depletion and in 5/8 models also increased sub-G1 populations suggestive of cell death. To identify the factors that played a role in the cell cycle arrest and cell death after combination treatment we performed Western (after 24h treatment) and qPCR (after 10h treatment) analyses of regulators of cell cycle and apoptosis (Figures 7 and 8). Western and qPCR results

suggested that the G1 arrest upon MDM2 inhibition was due to induction of p21 (CDKN1A). The p27 protein (CDKN1B) was induced by MEK inhibition in some of the models tested (see Westerns in Figure 7), which could potentially strengthen the cell cycle arrest in combination. MDM2 inhibition transcriptionally induced expression of the pro-apoptotic factors PUMA and BAX (Figure 7), and PUMA induction was also confirmed on Westerns (Figure 8). MEK inhibition showed elevated levels of the pro-apoptotic protein BIM (Figure 7), and transcriptionally induced expression of the pro-apoptotic factors BMF and NOXA1 (Figure 8). Together, induction of these genes and proteins in the drug combination could explain the increased PARP cleavage (cPARP) seen in the combination in all models but COLO-678 (Figure 7). cPARP is an indicator for the induction of apoptosis.

## *CONCLUSIONS*

In conclusion, the data suggested that the combined inhibition of MDM2 and MEK could regulate complementary sets of cell cycle arrest proteins (p21 and p27 induction) to induce G1 and/or G2 cell cycle arrest, and pro-apoptotic proteins (e.g. induction of BAX, BIM, and PUMA) to induce cell death by apoptosis. Combined inhibition of MDM2 and MEK in TP53 wild-type colorectal cancer may provide an effective therapeutic modality capable of improving responses compared to each of the single agents and lead to more durable responses in the clinic.

## Reference Example 2: The in vitro effect on proliferation of combining a MDM2 inhibitor and a MEK inhibitor with a Bcl2 inhibitor.

This study was designed to explore an in vitro effect on proliferation of combining the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A) and the MEK inhibitor trametinib (COMPOUND B) with the BCL-2/-XL inhibitor navitoclax (ABT-263) (COMPOUND C) in TP53 wild-type colorectal cancer cell lines.

## *METHODS*

COMPOUNDS A, B and C were dissolved in 100% DMSO (Sigma, Catalog number D2650) at concentrations of 20mM and stored at -20oC until use. Compounds were arrayed in drug master plates (Greiner, Catalog number 788876) and serially diluted 3-fold (7 steps) at 2000X concentration.

Colorectal cancer cell lines used for this study were obtained, cultured and processed from commercial vendors ATCC, and ECACC (Table 4). All cell line media were supplemented with 10% FBS (HyClone, Catalog number SH30071.03).

| Cell line | Driver mutations | Source | Source Cat Num | Medium | Medium Vendor | Medium Cat Num | #Cells | Treatment [h] |
|---|---|---|---|---|---|---|---|---|
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |

**Table 4.** Cell line information.

Cell lines were cultured in 37°C and 5% CO2 incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

To test the effect of the combination of COMPOUND A, COMPOUND B, and COMPOUND C on cell proliferation cells were plated in black 384-well microplates with clear bottom (Matrix/Thermo Scientific, Catalog number 4332) in 50ul media per well at cell densities between 500 and 1250 cells/well (Table 4) and allowed to incubate at 37 degrees, 5% CO2 for 24h. After 24h one 384-well plate per cell line was prepared for cell counting by microscopy (see below) without receiving treatment (= 'baseline'). The other cell plates were treated by transferring 25nl of the 2000X compound from drug master plates using an ATS acoustic liquid dispenser (ECD Biosystems) and resulting in a final 1X concentration. COMPOUND A was used over a final concentration range of 13nM - 10μM, COMPOUND B was used over a final concentration range of 13nM - 10uM, and COMPOUND C was used over a final concentration range of 0.4nM - 0.3 □M (7 1:3 dilution steps). In order to assess the effect of the triple combination all individual COMPOUNDS (A, B, C), all

three pair wise combinations (A+B, A+C, B+C), and the triple combination (A+B+C) were tested in the same experiment. Pair wise combinations and the triple combination were tested at a fixed ratio of 1:1 (for drug pairs) and 1: 1: 1 (for the drug triple) at each dilution resulting in 7 combination conditions per treatment. Additionally, negative controls (DMSO = 'vehicle') and positive controls (Staurosporine = killing cells, 7-point 1:2 dilution series for a dose range of 16nm - 1uM) were transferred as treatment controls, and compounds with no efficacy in the cell lines tested were used in combinations with COMPOUND A and COMPOUND B as combination controls (combinations that do not exceed the efficacy of the more efficacious single agent = 'non-interacting' combinations). After compound addition 50nl of 2mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 4), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy. Cells were fixed and permeabilised for 45 minutes in 4% PFA (Electron Microscopy Sciences, Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 μg/ml. Cells were washed three times with PBS and then plates were heat-sealed using a PlateLoc (Agilent Technologies) with aluminum seals (Agilent Technologies, Catalog number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figure 9 denoted as 'Normalized cell count' (= 'xnorm') on the y-axis. Synergistic combinations were identified using the highest single agent model (HSA) as null hypothesis (Berenbaum 1989). Excess over the HSA model predicts a functional connection between the inhibited targets (Lehar, Zimmermann et al. 2007, Lehar, Krueger et al. 2009). The model input were inhibition values per drug dose:

$$\mathbf{I = 1 - xnorm}$$

I: inhibition
xnorm: normalized cell count (median of three replicates)

At every dose point of the combination treatment the difference between the inhibition of the combination and the inhibition of the stronger of the two single agents was calculated (= model residuals). Similarly, to assess the synergy of triple combinations at every dose point the difference between the inhibition of the drug triple and the inhibition of the strongest drug pair was calculated. To favor combination effects at high inhibition the residuals were weighted with the observed inhibition at the same dose point. The overall combination score C of a drug combination is the sum of the weighted residuals over all concentrations:

$$\mathbf{C = \Sigma Conc\ (Idata * (Idata - Imodel))}$$

Idata: measured inhibition
Imodel: inhibition according to HSA null hypothesis

Robust combination z-scores (zC) were calculated as the ratio of the treatments' combination scores C and the median absolute deviation (mad) of non-interacting combinations:

$$zC = C / mad(Czero)$$

Czero: combination scores of non-interacting combinations

**zC is an indicator for the strength of the combination with:**

zC ≥ 3: synergy
3 > zC ≥ 2: weak synergy
zC < 2: no synergy

IC50 is the compound concentration that results in 50% of the cell counts relative to DMSO. IC50 calculations (see Table 5) were done using the DRC package in R (Ritz and Streibig 2005) and fitting a four-parameter log-logistic function to the data.

The compound's effect on apoptosis was determined by calculating the percentage of cells with activated Caspase 3/7 per treatment and time point relative to the raw cell counts (before subtraction of debris) (y-axis in Figure 10). Cell counts at time points that were not experimentally measured were obtained by regression analysis by fitting a linear model for log-transformed cell counts at day 0 and the end of the treatment (assuming exponential cell growth).

| Cell | IC50 COMPOUND A | IC50 COMPOUND B | IC50 COMPOUND C | Synergy z-score ($z_C$) |
|---|---|---|---|---|
| GP2d | 0.8 | >0.3 | >10 | 6.4 |
| HCT-116 | 0.1 | 0.038 | >10 | 3 |
| LS-180 | 0.7 | 0.018 | >10 | 1.4 |
| RKO | 1.2 | 0.021 | >10 | 1.4 |
| LoVo | 0.6 | 0.007 | >10 | 0.8 |

**Table 5.** Single agent IC50 values for each compound and synergy z-score measurements for the combination of COMPOUND A, COMPOUND B, and COMPOUND C.

***RESULTS***

In this report the efficacies of a MDM2 inhibitor (COMPOUND A), a MEK inhibitor (trametinib, COMPOUND B), and a BCL-2/-XL inhibitor (ABT-263, COMPOUND C) were assessed individually and in combination in a total of 5 TP53 wild type colorectal cancer cell lines. Four of the lines were KRAS mutant (GP2d, LS-180, HCT-116, LoVo), one line was BRAF mutant (RKO) (Table 4). COMPOUND A as single agent inhibited the growth of cell lines with sub-micromolar to micromolar IC50 values. COMPOUND B as single agent inhibited the growth of all but one cell line (GP2d) with nanomolar IC50 values, while COMPOUND C had no single agent efficacy (Figure 9 and Table 5). The triple combination (A+B+C) caused synergistic inhibition (according to the HSA model) over the drug pairs in 2/5 cell models tested (Table 5). In four of the lines (GP2d, HCT-116, RKO, LoVo) the triple combination showed stronger apoptosis (assessed by measuring Caspase 3/7 induction) compared to the pair wise combinations (Figure 10).

***CONCLUSIONS***

Collectively, combined inhibition of MDM2, MEK, and BCL-2/-XL in TP53 wild type CRC may provide an effective therapeutic modality capable of improving responses compared to each of the single agents and lead to more durable responses in the clinic.

In addition, a PI3K inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide was added to the combination (S)-1-(4-Chloro-phenyl)-7-isopropoxy-6-methoxy-2-(4-{methyl-[4-(4-methyl-3-oxo-piperazin-l-yl)-trans-cyclohexylmethyl]-amino}-phenyl)-1,4-dihydro-2H-isoquinolin-3-one (COMPOUND A) and the MEK inhibitor trametinib (COMPOUND B) with the BCL-2/-XL inhibitor navitoclax (ABT-263) (COMPOUND C) to form quadruple combination and together tested in 1 KRAS mutant cell line and found weakly synergistic (LS-180, combination z-score of 2.63) and strongly inducing apoptosis (maximum of 61%).

**Reference Example 3: The in vitro effect on proliferation of combining a MDM2 inhibitor and a MEK inhibitor with an EGFR inhibitor.**

This study was designed to explore an in vitro effect on proliferation of combining the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A) and the MEK inhibitor trametinib (COMPOUND B) with the EGFR inhibitor erlotinib (COMPOUND C) in TP53 wild-type colorectal cancer cell lines.

***METHODS***

COMPOUNDS A, B and C were dissolved in 100% DMSO (Sigma, Catalog number D2650) at concentrations of 20mM and stored at -20°C until use. Compounds were arrayed in drug master plates (Greiner, Catalog number 788876) and serially diluted 3-fold (7 steps) at 2000X concentration.

Colorectal cancer cell lines used for this study were obtained, cultured and processed from commercial vendors ATCC, and ECACC (Table 6). All cell line media were supplemented with 10% FBS (HyClone, Catalog number SH30071.03).

| Cell line | Driver mutations | Source | Source Cat Num | Medium | Medium Vendor | Medium Cat Num | #Cells | Treatment [h] |
|---|---|---|---|---|---|---|---|---|
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |

**Table 6.** Cell line information

Cell lines were cultured in 37°C and 5% $CO_2$ incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

To test the effect of the combination of COMPOUND A, COMPOUND B, and COMPOUND C on cell proliferation cells were plated in black 384-well microplates with clear bottom (Matrix/Thermo Scientific, Catalog number 4332) in 50ul media per well at cell densities between 500 and 1250 cells/well (Table 6) and allowed to incubate at 37 degrees, 5% $CO_2$ for 24h. After 24h one 384-well plate per cell line was prepared for cell counting by microscopy (see below) without receiving treatment (= 'baseline'). The other cell plates were treated by transferring 25nl of the 2000X compound from drug master plates using an ATS acoustic liquid dispenser (ECD Biosystems) and resulting in a final 1X concentration. COMPOUND A was used over a final concentration range of 13nM - 10uM, COMPOUND B was used over a final concentration range of 13nM - 10uM, and COMPOUND C was used over a final concentration range of 13nM - 10μM (7 1:3 dilution steps). In order to assess the effect of the triple combination all individual COMPOUNDS (A, B, C), all three pair wise combinations (A+B, A+C, B+C), and the triple combination (A+B+C) were tested in the same experiment. Pair wise combinations and the triple combination were tested at a fixed ratio of 1:1 (for drug pairs) and 1: 1: 1 (for the drug triple) at each dilution resulting in 7 combination conditions per treatment. Additionally, negative controls (DMSO = 'vehicle') and positive controls (Staurosporine = killing cells, 7-point 1:2 dilution series for a dose range of 16nm - 1uM) were transferred as treatment controls, and compounds with no efficacy in the cell lines tested were used in combinations with COMPOUND A and COMPOUND B as combination controls (combinations that do not exceed the efficacy of the more efficacious single agent = 'non-interacting' combinations). After compound addition 50nl of 2mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 6), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy. Cells were fixed and permeabilised for 45 minutes in 4% PFA (Electron Microscopy Sciences, Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 μg/ml. Cells were washed three times with PBS and then plates were heat-sealed using a PlateLoc (Agilent Technologies) with aluminum seals (Agilent Technologies, Catalog

number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figure 11 denoted as 'Normalized cell count' (= 'xnorm') on the y-axis. Synergistic combinations were identified using the highest single agent model (HSA) as null hypothesis (Berenbaum 1989). Excess over the HSA model predicts a functional connection between the inhibited targets (Lehar, Zimmermann et al. 2007, Lehar, Krueger et al. 2009). The model input were inhibition values per drug dose:

$$\mathbf{I = 1 - xnorm}$$

I: inhibition
xnorm: normalized cell count (median of three replicates)

At every dose point of the combination treatment the difference between the inhibition of the combination and the inhibition of the stronger of the two single agents was calculated (= model residuals). Similarly, to assess the synergy of triple combinations at every dose point the difference between the inhibition of the drug triple and the inhibition of the strongest drug pair was calculated. To favor combination effects at high inhibition the residuals were weighted with the observed inhibition at the same dose point. The overall combination score C of a drug combination is the sum of the weighted residuals over all concentrations:

$$\mathbf{C = \Sigma_{Conc} (I_{data} * (I_{data} - I_{model}))}$$

$I_{data}$: measured inhibition
$I_{model}$: inhibition according to HSA null hypothesis

Robust combination z-scores (zc) were calculated as the ratio of the treatments' combination scores C and the median absolute deviation (mad) of non-interacting combinations:

$$\mathbf{z_C = C / mad(C_{zero})}$$

$C_{zero}$: combination scores of non-interacting combinations

**$z_c$ is an indicator for the strength of the combination with:**

zc ≥ 3: synergy
3 > zc ≥ 2: weak synergy
zc < 2: no synergy

IC50 is the compound concentration that results in 50% of the cell counts relative to DMSO. IC50 calculations (see Table 7) were done using the DRC package in R (Ritz and Streibig 2005) and fitting a four-parameter log-logistic function to the data.

The compound's effect on apoptosis was determined by calculating the percentage of cells with activated Caspase 3/7 per treatment and time point relative to the raw cell counts (before subtraction of debris) (y-axis in Figure 12). Cell counts at time points that were not experimentally measured were obtained by regression analysis by fitting a linear model for log-transformed cell counts at day 0 and the end of the treatment (assuming exponential cell growth).

| Cell | IC50 COMPOUND A | IC50 COMPOUND B | IC50 COMPOUND C | Synergy z-score ($z_c$) |
|---|---|---|---|---|
| LoVo | 0.6 | 0.007 | 0.7 | 2.9 |
| LS-180 | 0.7 | 0.018 | >10 | 1.8 |
| GP2d | 0.8 | >0.3 | 8.9 | 0.8 |
| RKO | 1.2 | 0.021 | >10 | -1.3 |
| HCT-116 | 0.1 | 0.038 | >10 | -1.8 |

**Table 7.** Single agent IC50 values for each compound and synergy z-score measurements for the combination of COMPOUND A, COMPOUND B, and COMPOUND C.

***RESULTS***

In this report the efficacies of a MDM2 inhibitor (COMPOUND A), a MEK inhibitor (trametinib, COMPOUND B), and an EGFR inhibitor (erlotinib, COMPOUND C) were assessed individually and in combination in a total of 5 TP53 wild type colorectal cancer cell lines. Four of the lines were KRAS mutant (GP2d, LS-180, HCT-116, LoVo), one line was BRAF mutant (RKO) (Table 6). COMPOUND A as single agent inhibited the growth of cell lines with sub-micromolar to micromolar IC50 values. COMPOUND B as single agent inhibited the growth of all but one cell line (GP2d) with nanomolar IC50 values, while COMPOUND C had no single agent efficacy in 4/5 lines and a micromolar IC50 in the KRAS mutant LoVo (Figure 11 and Table 7). The triple combination (A+B+C) caused weak synergistic inhibition (according to the HSA model) over the drug pairs in the KRAS mutant model LoVo (Table 7). In this cell line the triple combination showed stronger apoptosis (assessed by measuring Caspase 3/7 induction) compared to the pair wise combinations (Figure 12).

***CONCLUSIONS***

Combined inhibition of MDM2, MEK, and EGFR in TP53 wild type CRC may provide an effective therapeutic modality capable of improving responses compared to each of the single agents and lead to more durable responses in the clinic.

**Reference Example 4: The in vitro effect on proliferation of combining a PI3K inhibitor and a MDM2 inhibitor with a Bcl2 inhibitor**

This study was designed to explore an in vitro effect on proliferation of combining the PIK3CA inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND A) and the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND B) with the BCL-2/-XL inhibitor navitoclax (ABT-263) (COMPOUND C) in TP53 wild-type colorectal cancer cell lines.

***METHODS***

COMPOUNDS A, B and C were dissolved in 100% DMSO (Sigma, Catalog number D2650) at concentrations of 20mM and stored at -20°C until use. Compounds were arrayed in drug master plates (Greiner, Catalog number 788876) and serially diluted 3-fold (7 steps) at 2000X concentration.

Colorectal cancer cell lines used for this study were obtained, cultured and processed from commercial vendors ATCC, and ECACC (Table 8). All cell line media were supplemented with 10% FBS (HyClone, Catalog number SH30071.03).

| Cell line | Driver mutations | Source | Source Cat Num | Medium | Medium Vendor | Medium Cat Num | #Cells | Treatment [h] |
|---|---|---|---|---|---|---|---|---|
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |

**Table 8.** Cell line information

Cell lines were cultured in 37°C and 5% $CO_2$ incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

To test the effect of the combination of COMPOUND A, COMPOUND B, and COMPOUND C on cell proliferation cells were plated in black 384-well microplates with clear bottom (Matrix/Thermo Scientific, Catalog number 4332) in 50ul media per well at cell densities between 500 and 1250 cells/well (Table 8) and allowed to incubate at 37 degrees, 5% $CO_2$ for 24h. After 24h one 384-well plate per cell line was prepared for cell counting by microscopy (see below) without receiving treatment (= 'baseline'). The other cell plates were treated by transferring 25nl of the 2000X compound from drug master plates using an ATS acoustic liquid dispenser (ECD Biosystems) and resulting in a final 1X concentration. COMPOUND A was used over a final concentration range of 13nM - 10uM, COMPOUND B was used over a final concentration range of 13nM - 10uM, and COMPOUND C was used over a final concentration range of 13nM - 10μM (7 1:3 dilution steps). In order to assess the effect of the triple combination all individual COMPOUNDS (A, B, C), all three pair wise combinations (A+B, A+C, B+C), and the triple combination (A+B+C) were tested in the same experiment. Pair wise combinations and the triple combination were tested at a fixed ratio of 1:1 (for drug pairs) and 1: 1: 1 (for the drug triple) at each dilution resulting in 7 combination conditions per treatment. Additionally, negative controls (DMSO = 'vehicle') and positive controls (Staurosporine = killing cells, 7-point 1:2 dilution series for a dose range of 16nm - 1uM) were transferred as treatment controls, and compounds with no efficacy in the cell lines tested were used in combinations with COMPOUND A and COMPOUND B as combination controls (combinations that do not exceed the efficacy of the more efficacious single agent = 'non-interacting' combinations). After compound addition 50nl of 2mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 8), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy. Cells were fixed and permeabilised for 45 minutes in 4% PFA (Electron Microscopy Sciences, Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 μg/ml. Cells were washed three times with PBS and then plates were heat-sealed using a PlateLoc (Agilent Technologies) with aluminum seals (Agilent Technologies, Catalog number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figure 13 denoted as 'Normalized cell count' (= 'xnorm') on the y-axis. Synergistic combinations were identified using the highest single agent model (HSA) as null hypothesis (Berenbaum 1989). Excess over the HSA model predicts a functional connection between the inhibited targets (Lehar, Zimmermann et al. 2007, Lehar, Krueger et al. 2009). The model input were inhibition values per drug dose:

$$\mathbf{I = 1 - xnorm}$$

I: inhibition
xnorm: normalized cell count (median of three replicates)

At every dose point of the combination treatment the difference between the inhibition of the combination and

the inhibition of the stronger of the two single agents was calculated (= model residuals). Similarly, to assess the synergy of triple combinations at every dose point the difference between the inhibition of the drug triple and the inhibition of the strongest drug pair was calculated. To favor combination effects at high inhibition the residuals were weighted with the observed inhibition at the same dose point. The overall combination score C of a drug combination is the sum of the weighted residuals over all concentrations:

$$C = \Sigma_{Conc} (I_{data} * (I_{data} - I_{model}))$$

$I_{data}$: measured inhibition
$I_{model}$: inhibition according to HSA null hypothesis

Robust combination z-scores (zc) were calculated as the ratio of the treatments' combination scores C and the median absolute deviation (mad) of non-interacting combinations:

$$z_C = C / mad(C_{zero})$$

$C_{zero}$: combination scores of non-interacting combinations

**$z_c$ is an indicator for the strength of the combination with:**

zc ≥ 3: synergy
3 > zc ≥ 2: weak synergy
zc < 2: no synergy

IC50 is the compound concentration that results in 50% of the cell counts relative to DMSO. IC50 calculations (see Table 9) were done using the DRC package in R (Ritz and Streibig 2005) and fitting a four-parameter log-logistic function to the data.

The compound's effect on apoptosis was determined by calculating the percentage of cells with activated Caspase 3/7 per treatment and time point relative to the raw cell counts (before subtraction of debris) (y-axis in Figure 14). Cell counts at time points that were not experimentally measured were obtained by regression analysis by fitting a linear model for log-transformed cell counts at day 0 and the end of the treatment (assuming exponential cell growth).

| Cell | IC50 COMPOUND A | IC50 COMPOUND B | IC50 COMPOUND C | Synergy z-score ($z_c$) |
|---|---|---|---|---|
| GP2d | 0.5 | 0.8 | >10 | 7.4 |
| HCT-116 | 9.8 | 0.1 | >10 | 4.4 |
| LoVo | >10 | 0.6 | >10 | 1.9 |
| RKO | 3.9 | 1.2 | >10 | 1.8 |
| LS-180 | >10 | 0.7 | >10 | 1.5 |

**Table 9.** Single agent IC50 values for each compound and synergy z-score measurements for the combination of COMPOUND A, COMPOUND B, and COMPOUND C.

## *RESULTS*

In this report the efficacies of a PIK3CA inhibitor (BYL719, COMPOUND A), a MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND B), and a BCL-2/-XL inhibitor (ABT-263, COMPOUND C) were assessed individually and in combination in a total of 5 TP53 wild type colorectal cancer cell lines. Four of the lines were KRAS mutant (GP2d, LS-180, HCT-116, LoVo), one line was BRAF mutant (RKO). COMPOUND A as single agent inhibited the growth of 2 of the cell lines with micromolar IC50 values, and was active only at the highest dose (10uM) in the 3 other lines (Figure 13 and Table 9). COMPOUND B as single agent inhibited the growth of cell lines with sub-micromolar to micromolar IC50 values, while COMPOUND C had no single agent efficacy (Figure 13 and Table 9). The triple combination (A+B+C) caused synergistic inhibition (according to the HSA model) over the drug pairs in 2/5 cell models tested (Table 9). In four of the lines (HCT-116, LoVo, RKO, LS-180) the triple combination showed stronger

apoptosis (assessed by measuring Caspase 3/7 induction) compared to the pair wise combinations (Figure 14).

## *CONCLUSIONS*

Collectively, combined inhibition of PIK3CA, MDM2, and BCL-2/-XL in TP53 wild type CRC may provide an effective therapeutic modality capable of improving responses compared to each of the single agents and lead to more durable responses in the clinic.

## Reference Example 5: The in vitro effect on proliferation of combining a MDM2 inhibitor and a Bcl2 inhibitor

This study was designed to explore an in vitro effect on proliferation of combining the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one (COMPOUND A) and the BCL-2/-XL inhibitor navitoclax (ABT-263) (COMPOUND B) in TP53 wild-type colorectal cancer cell lines.

## *METHODS*

COMPOUNDS A and B were dissolved in 100% DMSO (Sigma, Catalog number D2650) at concentrations of 20mM and stored at -20°C until use. Compounds were arrayed in drug master plates (Greiner, Catalog number 788876) and serially diluted 3-fold (7 steps) at 2000X concentration.

Colorectal cancer cell lines used for this study were obtained, cultured and processed from the commercial vendors ATCC, and ECACC (Table 10). All cell line media were supplemented with 10% FBS (HyClone, Catalog number SH30071.03).

| Cell line | Driver mutations | Source | Source Cat Num | Medium | Medium Vendor | Medium Cat Num | #Cells | Treatment [h] |
|---|---|---|---|---|---|---|---|---|
| HCT-116 | KRAS, PIK3CA | ATCC | CCL-247 | McCoy's 5A | ATCC | 30-2007 | 500 | 72 |
| LS-180 | KRAS, PIK3CA | ATCC | CCL-187 | EMEM | ATCC | 30-2003 | 800 | 72 |
| GP2d | KRAS, PIK3CA | ECACC | 95090714 | DMEM | ATCC | 30-2002 | 900 | 72 |
| LoVo | KRAS | ATCC | CCL-229 | F-12K | ATCC | 30-2004 | 1250 | 96 |
| RKO | BRAF,PIK3CA | ATCC | CRL-2577 | EMEM | ATCC | 30-2003 | 500 | 72 |

**Table 10.** Cell line information

Cell lines were cultured in 37°C and 5% $CO_2$ incubator and expanded in T-75 flasks. In all cases cells were thawed from frozen stocks, expanded through ≥1 passage using 1:3 dilutions, counted and assessed for viability using a ViCell counter (Beckman-Coulter) prior to plating. To split and expand cell lines, cells were dislodged from flasks using 0.25% Trypsin-EDTA (GIBCO, Catalog number 25200). All cell lines were determined to be free of mycoplasma contamination as determined by a PCR detection methodology performed at Idexx Radii (Columbia, MO, USA) and correctly identified by detection of a panel of SNPs.

To test the effect of the combination of COMPOUND A and COMPOUND B on cell proliferation cells were plated in black 384-well microplates with clear bottom (Matrix/Thermo Scientific, Catalog number 4332) in 50ul media per well at cell densities between 500 and 1250 cells/well (Table 10) and allowed to incubate at 37 degrees, 5% $CO_2$ for 24h. After 24h one 384-well plate per cell line was prepared for cell counting by microscopy (see below) without receiving treatment (= 'baseline'). The other cell plates were treated by transferring 25nl of the 2000X compound from drug master plates using an ATS acoustic liquid dispenser (ECD Biosystems) and resulting in a final 1X concentration. COMPOUND A was used over a final concentration range of 13nM - 10uM, and COMPOUND B was used over a final concentration range of 13nM - 10μM (7 1:3 dilution steps). For the combination of COMPOUND A with COMPOUND B the single agents were combined at a fixed ratio of 1:1 at each dilution resulting in 7 combination treatments. Additionally, negative controls (DMSO = 'vehicle') and positive controls (Staurosporine = killing cells, 7-point 1:2 dilution series for a dose range of 16nm - 1uM) were transferred as treatment controls, and compounds with no efficacy in the cell lines tested were used in combinations with COMPOUND A and COMPOUND B as combination controls (combinations that do not exceed the efficacy of the more efficacious single agent = 'non-interacting' combinations). After compound addition 50nl of 2mM CellEvent Caspase-3/7 Green Detection Reagent (ThermoFisher, Catalog number C10423) were added to one of the three replicates using the HP D300 Digital Dispenser (Tecan). Caspase 3/7 induction was measured as a proxy for apoptosis induced by the treatments. Cells were treated for 72h to 96h depending on their doubling time (Table 10), and Caspase 3/7 activation was measured every 24h by microscopy using an InCell Analyzer 2000 (GE Healthcare) equipped with a 4X objective and FITC excitation/emission filters. At the end of the treatment cells were prepared for cell counting by microscopy. Cells were fixed and permeabilised for 45 minutes in 4% PFA (Electron Microscopy Sciences,

Catalog number 15714), 0.12% TX-100 (Electron Microscopy Sciences, Catalog number 22140) in PBS (Boston Bioproducts, Catalog number BM-220). After washing cells three times with PBS their DNA was stained for 30 minutes with Hoechst 33342 (ThermoFisher, Catalog number H3570) at a final concentration of 4 μg/ml. Cells were washed three times with PBS and then plates were heat-sealed using a PlateLoc (Agilent Technologies) with aluminum seals (Agilent Technologies, Catalog number 06644-001) and stored at 4°C until imaging. All cells per well/treatment were captured in a single image by fluorescence microscopy using an InCell Analyzer 2000 (GE Healthcare) equipped with a 4X objective and DAPI excitation/emission filters.

Images were analyzed after adapting previously described methods (Horn, Sandmann et al. 2011) and using the Bioconductor package EBImage in R (Pau, Fuchs et al. 2010). Objects in both channels, DAPI (for Hoechst/DNA) and FITC (for Caspase 3/7), were segmented separately by adaptive thresholding and counted. A threshold for Caspase 3/7 positive objects was defined manually per cell line after comparing negative controls (DMSO) and positive controls (Staurosporine). By analyzing 17 additional object/nuclei features in the DNA channel (shape and intensity features) debris/fragmented nuclei were identified. To this end per cell line the distributions of the additional features between positive controls (Staurosporine) and negative controls (DMSO) were compared manually. Features that could differentiate between the conditions (e.g. a shift in the distribution of a feature measurement comparing DMSO with Staurosporine) where used to define the 'debris' population versus the population of 'viable' nuclei. The debris counts were subtracted from raw nuclei counts. The resulting nuclei number was used as measure of cell proliferation ('cell count').

The compound's effect on cell proliferation was calculated from the cell counts of the treatments relative to the cell counts of the negative control (DMSO), in Figure 15 denoted as 'Normalized cell count' (= 'xnorm') on the y-axis. Synergistic combinations were identified using the highest single agent model (HSA) as null hypothesis (Berenbaum 1989). Excess over the HSA model predicts a functional connection between the inhibited targets (Lehar, Zimmermann et al. 2007, Lehar, Krueger et al. 2009). The model input were inhibition values per drug dose:

$$\mathbf{I = 1 - xnorm}$$

I: inhibition
xnorm: normalized cell count (median of three replicates)

At every dose point of the combination treatment the difference between the inhibition of the combination and the inhibition of the stronger of the two single agents was calculated (= model residuals). To favor combination effects at high inhibition the residuals were weighted with the observed inhibition at the same dose point. The overall combination score C of a drug combination is the sum of the weighted residuals over all concentrations:

$$C = \Sigma_{Conc} (I_{data} * (I_{data} - I_{model}))$$

$I_{data}$: measured inhibition
$I_{model}$: inhibition according to HSA null hypothesis

Robust combination z-scores (zc) were calculated as the ratio of the treatments' combination scores C and the median absolute deviation (mad) of non-interacting combinations:

$$z_C = C / mad(C_{zero})$$

$C_{zero}$: combination scores of non-interacting combinations

**$z_c$ is an indicator for the strength of the combination with:**

zc ≥ 3: synergy
3 > zc ≥ 2: weak synergy
zc < 2: no synergy

IC50 is the compound concentration that results in 50% of the cell counts relative to DMSO. IC50 calculations (see Table 11) were done using the DRC package in R (Ritz and Streibig 2005) and fitting a four-parameter log-logistic function to the data.

The compound's effect on apoptosis was determined by calculating the percentage of cells with activated Caspase 3/7 per treatment and time point relative to the raw cell counts (before subtraction of debris) (y-axis in Figure

16). Cell counts at time points that were not experimentally measured were obtained by regression analysis by fitting a linear model for log-transformed cell counts at day 0 and the end of the treatment (assuming exponential cell growth).

| Cell | IC50 COMPOUND A | IC50 COMPOUND B | Synergy z-score ($z_C$) |
|---|---|---|---|
| GP2d | 0.8 | >10 | 14.6 |
| HCT-116 | 0.1 | >10 | 6.4 |
| LoVo | 0.6 | >10 | 3.3 |
| LS-180 | 0.7 | >10 | 2.3 |
| RKO | 1.2 | >10 | 1.6 |

**Table 11.** Single agent IC50 values for each compound and synergy z-score measurements for the combination of COMPOUND A and COMPOUND B.

***RESULTS***

In this report the efficacies of a MDM2 inhibitor (COMPOUND A) and a BCL-2/- XL inhibitor (COMPOUND B) were assessed individually and in combination in a total of 5 TP53 wild type colorectal cancer cell lines. Four of the lines were KRAS mutant (GP2d, LS-180, HCT-116, LoVo), one line was BRAF mutant (RKO), and four of the lines were also mutant for PIK3CA (GP2d, RKO, LS-180, HCT-116) (Table 10). COMPOUND A as single agent inhibited the growth of all cell lines with sub-micromolar to micromolar IC50 values (Figure 15 and Table 11). COMPOUND B had no single agent efficacy (Figure 15 and Table 11). The combination treatment caused synergistic inhibition (according to the HSA model) in 3/5 cell models, and weakly synergistic inhibition in 1 more model (Table 11). The combination also showed stronger induction of apoptosis (assessed by measuring Caspase 3/7 induction) compared to the single agents (Figure 16), with the strongest inductions seen in GP2d, HCT-116, and LoVo.

***CONCLUSIONS***

Combined inhibition of MDM2 and BCL-2/-XL in TP53 wild-type, KRAS and BRAF mutant colorectal cancer may provide an effective therapeutic modality capable of improving responses compared to each of the single agents and lead to more durable responses in the clinic.

**Reference Example 6: The in vitro effect on proliferation of combining a MDM2 inhibitor and a MEK inhibitor and a Bcl2 inhibitor**

Similarly as described in previous examples, a triple combination of (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one, trametinib and RAF265 was tested in 4 KRAS mutant models (HCT-116, GP2d, LoVo, and LS-180). The combination synergized in 2/4 models (HCT-116, GP2d, with combination z-scores of 9.2 and 5.5, respectively), and weakly synergized in 1/4 (LoVo, with combination z-score of 2.8). No synergy was observed in LS-180 model (combination z-score of 0.2). Further the triple combination showed stronger induction of apoptosis when compared to the drug pairs (assessed by measuring Caspase 3/7 activation) in Gp2D and LoVo models (in GP2d a maximum of 67% apoptosis, in LoVo 83%). Observed apoptosis levels caused by the triple combination in HCT-116 and LS-180 were 49% and 15%, respectively.

**Reference Example 7: The in vitro effect on proliferation of combining a MDM2 inhibitor and a MEK inhibitor and a BRAF inhibitor**

Similarly as described in previous examples, a triple combination of (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one, trametinib and dabrafenib was tested in 1 BRAF mutant model (RKO). We found the triple combination to be synergistic (combination z-score of 3.5), and showed stronger (but overall weak, with a maximum of 11%) induction of apoptosis when compared to the drug pairs (assessed by measuring Caspase 3/7 activation).

**Reference Example 8: The in vitro effect on proliferation of combining a MDM2 inhibitor and a BRAF inhibitor and a Bcl2 inhibitor, optionally together with a PI3K inhibitor or a cMET inhibitor**

Similarly as described in previous examples, a triple combination of (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one, RAF265 and navitoclax (ABT-263) was tested in 4 KRAS mutant models (HCT-116, GP2d, LoVo, and LS-180). The combination synergized in 1/4 models (GP2d, combination z-score of 5) and showed stronger induction of apoptosis when compared to the drug pairs (assessed by measuring Caspase 3/7 activation) in all 4 models tested (in GP2d and LoVo a maximum of 100% apoptosis, in HCT-116 64%, and in LS-180 32%). No synergy was observed in HCT-116, LS-180, and LoVo (combination z-scores of 1.3, 1.2, and 0.5, respectively).

In the same category, a triple combination of (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one, dabrafenib and navitoclax (ABT-263) was tested in 1 BRAF mutant model (RKO). We found the triple combination to be synergistic (combination z-score of 3), and showing strong induction of apoptosis (maximum of 100%), while all pairs showed only weak to no induction of apoptosis (assessed by measuring Caspase 3/7 activation).

Furthermore, two quadruple combinations were tested that comprised the MDM2 inhibitor (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one, BRAF inhibitor dabrafenib and BCl inhibitor navitoclax (ABT-263). The first was with a PI3K inhibitor (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide and found weakly synergistic in 1 BRAF mutant cell line (RKO, combination z-score of 2), and strongly inducing apoptosis (maximum of 79%). The second included a cMET inhibitor PF-04217903 (Pfizer. Once tested it was found to work weakly synergistic compared to triple combinations in 1 BRAF mutant cell line (RKO, combination z-score of 4.4), and strongly inducing apoptosis (maximum of 77%).

**Reference Example 9: The in vitro effect on proliferation of combining a MDM2 inhibitor and a MEK inhibitor and a** CDK4/6 **inhibitor or paclitaxel**

Adding a CDK4/6 inhibitor (specifically 7-cyclopentyl-N,N-dimethyl-2-((5-(piperazin-1-yl)pyridin-2-yl)amino)-7H-pyrrolo[2,3-d]pyrimidine-6-carboxamide) to a combination of (S)-1-(4-Chloro-phenyl)-7-isopropoxy-6-methoxy-2-(4-{methyl-[4-(4-methyl-3-oxo-piperazin-1-yl)-trans-cyclohexylmethyl]-amino}-phenyl)-1,4-dihydro-2H-isoquinolin-3-one and trametinib led to synergistic effect in 2/5 models tested (HCT-116, LoVo; z-scores of 5.1, 3.3 respectively), and acted weakly synergistic in 2/5 models (LS-180, RKO; z-scores of 2, 2.8 respectively). No synergy was observed in GP2d (z-score of 1.7). No induction of apoptosis was observed.

Adding paclitaxel (standard of care treatment) to the combination led to synergistic effect in 1/5 models tested (GP2d, z-score of 4.3), and weakly synergistic effect in 3/5 models tested (HCT-116, LoVo, LS-180; z-scores of 2.4, 2, and 2.4 respectively). No synergy was observed in RKO (z-score 1.1).Furthermore, the combination strongly induced apoptosis (GP2d: 100%, HCT-116: 59%, LoVo: 61%, LS-180: 20%, and RKO 65%).

**Reference Example 10: The in vivo effect on proliferation of combining an MDM2 inhibitor with a MEK inhibitor and a BCL-2/-XL inhibitor in a TP53 wild-type model of colorectal cancer**

Generation of tumor-bearing mice and treatment

All animal experiments were done in strict adherence to the Swiss law for animal protection. Female Crl:NU(NCr)-Foxnlnu mice were purchased from Charles River Laboratories International Inc (Germany) and kept in a pathogen-controlled environment. Subcutaneous tumors of HCT-116 (KRAS mutant, PIK3CA mutant, p53 wild-type) were induced by concentrating 3 million cells in 100 μl of PBS and injecting them in the right flank of nude mice. The mice were randomly grouped, and treatment was started when the tumor size reached 50 to 250 mm3 Each cohort included 8 mice Tumor sizes were monitored three times weekly, and volumes were calculated with the following formula: (mm3) = length × width2 × 0.5.

The MDM2 inhibitor (S)-1-(4-Chloro-phenyl)-7-isopropoxy-6-methoxy-2-(4-{methyl-[4-(4-methyl-3-oxo-piperazin-l-yl)-trans-cyclohexylmethyl]-amino}-phenyl)-1,4-dihydro-2H-isoquinolin-3-one (COMPOUND A), the MEK inhibitor trametinib (COMPOUND B), and the BCL-2/-XL inhibitor ABT-263 (COMPOUND C) in powder form were stored at +4°C. COMPOUND A was dissolved in 0.5% hydroxypropyl methylcellulose, COMPOUND B was dissolved in 1% carboxymethylcellulose containing 0.5% Tween-80% in distilled water (pH7.6-8.0), and COMPOUND C was dissolved in Microemulsion pre-concentrate 5. All drugs were dosed orally using 5-10 ml/kg. COMPOUND A was administered three times a week (3qw) at 100 mg/kg. COMPOUND B and COMPOUND C were administered daily (q24h) at 0.3 and 100 mg/kg, respectively. The combination dosing schedule and dosage were the same as the single reagents.

Six treatment cohorts (G1-G6) were tested:

- G1: vehicle (DMSO)
- G2: COMPOUND C
- G3: COMPOUND A => after 9 days treatment COMPOUND C was added
- G4: COMPOUND B => after 9 days treatment COMPOUND C was added
- G5: COMPOUND A + COMPOUND B => after 9 days treatment COMPOUND C was added
- G6: COMPOUND A + COMPOUND B + COMPOUND C

Statistical analysis

For each tumor at each time-point the size was normalized to the size before the start of the treatment to obtain the "% Change tumor volume" (Figure 17-18, y-axis). For Figure 17 at each time point the mean size of all tumors per cohort was calculated, and the error of the size using the standard error of the mean (SEM). For Figure 18 p-values were calculated using a one-tailed t test.

Results

In a xenograft model of HCT-116 cells the combination treatment of COMPOUND A and trametinib (G5) was significantly better (stable disease) when compared to each of the single agent treatments (G3-G4 showing progressive disease), and the triple combination of COMPOUND A, trametinib, and ABT-263 (G6) led to marked tumor regression and had a significantly better response compared to G5 (Figure 17 and Figure 18A). Figure 17 shows summarized survival curves, and Figure 18 shows waterfall plots at day 9 and day 19 after start of the treatments. Sequential addition of ABT-263 after 9 days to single agent COMPOUND A had no additional benefit, while it stopped tumor progression when added to single agent trametinib. ABT-263 led to marked tumor regressions when added to the combination of COMPOUND A and trametinib, and at day 19 the responses of concomitant and sequential treatments were indistinguishable (Figure 18B).

REFERENCES

Arrington, A. K., E. L. Heinrich, W. Lee, M. Duldulao, S. Patel, J. Sanchez, J. Garcia-Aguilar and J. Kim (2012). "Prognostic and predictive roles of KRAS mutation in colorectal cancer." Int J Mol Sci 13(10): 12153-12168.
Berenbaum, M. C. (1989). "What is synergy?" Pharmacol Rev 41(2): 93-141.
Bozic, I., J. G. Reiter, B. Allen, T. Antal, K. Chatterjee, P. Shah, Y. S. Moon, A. Yaqubie, N. Kelly, D. T. Le, E. J. Lipson, P. B. Chapman, L. A. Diaz, Jr., B. Vogelstein and M. A. Nowak (2013). "Evolutionary dynamics of cancer in response to targeted combination therapy." Elife 2: e00747.
Brana, I. and L. L. Siu (2012). "Clinical development of phosphatidylinositol 3-kinase inhibitors for cancer treatment." BMC Med 10: 161.
Cancer Genome Atlas, N. (2012). "Comprehensive molecular characterization of human colon and rectal cancer." Nature 487(7407): 330-337.
Chandarlapaty, S. (2012). "Negative feedback and adaptive resistance to the targeted therapy of cancer." Cancer Discov 2(4): 311-319.
Chapman, P. B., D. B. Solit and N. Rosen (2014). "Combination of RAF and MEK inhibition for the treatment of BRAF-mutated melanoma: feedback is not encouraged." Cancer Cell 26(5): 603-604.
Chatterjee, M. S., J. E. Purvis, L. F. Brass and S. L. Diamond (2010). "Pairwise agonist scanning predicts cellular signaling responses to combinatorial stimuli." Nat Biotechnol 28(7): 727-732.
Chou, T. C. and P. Talalay (1981). "Generalized equations for the analysis of inhibitions of Michaelis-Menten and higher-order kinetic systems with two or more mutually exclusive and nonexclusive inhibitors." Eur J Biochem 115(1): 207-216.
DeVita, V. T., Jr. (1975). "Single agent versus combination chemotherapy." CA Cancer J Clin 25(3): 152-158.
Doebele, R. C., A. B. Pilling, D. L. Aisner, T. G. Kutateladze, A. T. Le, A. J. Weickhardt, K. L. Kondo, D. J. Linderman, L. E. Heasley, W. A. Franklin, M. Varella-Garcia and D. R. Camidge (2012). "Mechanisms of resistance to crizotinib in patients with ALK gene rearranged non-small cell lung cancer." Clin Cancer Res 18(5): 1472-1482.

Druker, B. J. (2008). "Translation of the Philadelphia chromosome into therapy for CML." Blood 112(13): 4808-4817.

Duncan, J. S., M. C. Whittle, K. Nakamura, A. N. Abell, A. A. Midland, J. S. Zawistowski, N. L. Johnson, D. A. Granger, N. V. Jordan, D. B. Darr, J. Usary, P. F. Kuan, D. M. Smalley, B. Major, X. He, K. A. Hoadley, B. Zhou, N. E. Sharpless, C. M. Perou, W. Y. Kim, S. M. Gomez, X. Chen, J. Jin, S. V. Frye, H. S. Earp, L. M. Graves and G. L. Johnson (2012). "Dynamic reprogramming of the kinome in response to targeted MEK inhibition in triple-negative breast cancer." Cell 149(2): 307-321.

Faber, A. C., E. M. Coffee, C. Costa, A. Dastur, H. Ebi, A. N. Hata, A. T. Yeo, E. J. Edelman, Y. Song, A. T. Tam, J. L. Boisvert, R. J. Milano, J. Roper, D. P. Kodack, R. K. Jain, R. B. Corcoran, M. N. Rivera, S. Ramaswamy, K. E. Hung, C. H. Benes and J. A. Engelman (2014). "mTOR inhibition specifically sensitizes colorectal cancers with KRAS or BRAF mutations to BCL-2/BCL-XL inhibition by suppressing MCL-1." Cancer Discov 4(1): 42-52.

Fearon, E. R. (2011). "Molecular genetics of colorectal cancer." Annu Rev Pathol 6: 479-507.

Horn, T., T. Sandmann, B. Fischer, E. Axelsson, W. Huber and M. Boutros (2011). "Mapping of signaling networks through synthetic genetic interaction analysis by RNAi." Nat Methods 8(4): 341-346.

Ji, Z., C. N. Njauw, M. Taylor, V. Neel, K. T. Flaherty and H. Tsao (2012). "p53 rescue through HDM2 antagonism suppresses melanoma growth and potentiates MEK inhibition." J Invest Dermatol 132(2): 356-364.

Katayama, R., A. T. Shaw, T. M. Khan, M. Mino-Kenudson, B. J. Solomon, B. Halmos, N. A. Jessop, J. C. Wain, A. T. Yeo, C. Benes, L. Drew, J. C. Saeh, K. Crosby, L. V. Sequist, A. J. Iafrate and J. A. Engelman (2012). "Mechanisms of acquired crizotinib resistance in ALK-rearranged lung Cancers." Sci Transl Med 4(120): 120ra117.

Lehar, J., A. Krueger, G. Zimmermann and A. Borisy (2008). "High-order combination effects and biological robustness." Mol Syst Biol 4: 215.

Lito, P., N. Rosen and D. B. Solit (2013). "Tumor adaptation and resistance to RAF inhibitors." Nat Med 19(11): 1401-1409.

Pau, G., F. Fuchs, O. Sklyar, M. Boutros and W. Huber (2010). "EBImage--an R package for image processing with applications to cellular phenotypes." Bioinformatics 26(7): 979-981.

Porter, K., A. Babiker, K. Bhaskaran, J. Darbyshire, P. Pezzotti, K. Porter, A. S. Walker and C. Collaboration (2003). "Determinants of survival following HIV-1 seroconversion after the introduction of HAART." Lancet 362(9392): 1267-1274.

Robert, C., B. Karaszewska, J. Schachter, P. Rutkowski, A. Mackiewicz, D. Stroiakovski, M. Lichinitser, R. Dummer, F. Grange, L. Mortier, V. Chiarion-Sileni, K. Drucis, I. Krajsova, A. Hauschild, P. Lorigan, P. Wolter, G. V. Long, K. Flaherty, P. Nathan, A. Ribas, A. M. Martin, P. Sun, W. Crist, J. Legos, S. D. Rubin, S. M. Little and D. Schadendorf (2015). "Improved overall survival in melanoma with combined dabrafenib and trametinib." N Engl J Med 372(1): 30-39.

Safaee Ardekani, G., S. M. Jafarnejad, L. Tan, A. Saeedi and G. Li (2012). "The prognostic value of BRAF mutation in colorectal cancer and melanoma: a systematic review and meta-analysis." PLoS One 7(10): e47054.

Shoemaker, R. H. (2006). "The NCI60 human tumour cell line anticancer drug screen." Nat Rev Cancer 6(10): 813-823.

Solit, D. B. and N. Rosen (2014). "Towards a unified model of RAF inhibitor resistance." Cancer Discov 4(1): 27-30.

Sullivan, R. J. and K. T. Flaherty (2013). "Resistance to BRAF-targeted therapy in melanoma." Eur J Cancer 49(6): 1297-1304.

Turner, N. C., J. Ro, F. Andre, S. Loi, S. Verma, H. Iwata, N. Harbeck, S. Loibl, C. Huang Bartlett, K. Zhang, C. Giorgetti, S. Randolph, M. Koehler and M. Cristofanilli (2015). "Palbociclib in Hormone-Receptor-Positive Advanced Breast Cancer." N Engl J Med.

## Claims

1. A pharmaceutical combination comprising the MDM2 inhibitor (6S)-5-(5-Chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-d]imidazol-4(1H)-one (HDM201), or a pharmaceutically acceptable salt thereof, and the Bcl2 inhibitor ABT-199, or a pharmaceutically acceptable salt thereof.

2. The pharmaceutical combination according to claim 1 further comprising a MEK inhibitor selected from the group consisting of trametinib, 6-(4-bromo-2-fluorophenylamino)-7-fluoro-3-methyl-3H-benzoimidazole-5-carboxylic acid (2-hydroxyethoxy)-amide, (*S*)-5-fluoro-2-(2-fluoro-4-(methylthio)phenylamino)-*N*-(2-hydroxypropoxy)-1-methyl-6-oxo-1,6-dihydropyridine-3-carboxamide, PD0325901, PD-184352, RDEA119, XL518, AS-701255, AS-701173, AS703026, RDEA436, E6201, RO4987655, RG7167, and RG7420 or a pharmaceutically acceptable salt thereof.

3. The pharmaceutical combination according to any one of claims 1 to 2, wherein the combination further comprises an EGFR inhibitor, a PI3K inhibitor, a BRAF inhibitor, a CDK4/6 inhibitor or paclitaxel.

4. The pharmaceutical combination according to claim 1 or claim 2, wherein the combination further comprises a BRAF inhibitor, and optionally yet further comprises a cMET inibitor.

5. HDM201, or a pharmaceutically acceptable salt thereof for use in the treatment of cancer, wherein the treatment further comprises administration of ABT-199, or a pharmaceutically acceptable salt thereof.

6. ABT-199 or a pharmaceutically acceptable salt thereof for use in the treatment of cancer, wherein the treatment further comprises administration of HDM201, or a pharmaceutically acceptable salt thereof.

7. The HDM201 for use according to claim 5 or the ABT-199 for use according to claim 6, wherein the cancer is selected from the group consisting of a benign or malignant tumor of the lung (including small cell lung cancer and non-small-cell lung cancer), bronchus, prostate, breast (including sporadic breast cancers and sufferers of Cowden disease), pancreas, gastrointestinal tract, colon, rectum, colon carcinoma, colorectal cancer, thyroid, liver, biliary tract, intrahepatic bile duct, hepatocellular, adrenal gland, stomach, gastric, glioma, glioblastoma, endometrial, kidney, renal pelvis, bladder, uterus, cervix, vagina, ovary, multiple myeloma, esophagus, neck or head, brain, oral cavity and pharynx, larynx, small intestine, a melanoma, villous colon adenoma, a sarcoma, a neoplasia, a neoplasia of epithelial character, a mammary carcinoma, basal cell carcinoma, squamous cell carcinoma, actinic keratosis, polycythemia vera, essential thrombocythemia, a leukemia (including acute myelogenous leukemia, chronic myelogenous leukemia, lymphocytic leukemia, and myeloid leukemia), a lymphoma (including non-Hodgkin lymphoma and Hodgkin's lymphoma), myelofibrosis with myeloid metaplasia, Waldenstroem disease, and Barret's adenocarcinoma.

8. The HDM201for use according to claim 7 or the ABT-199 for use according to claim 7, wherein the cancer is a colorectal cancer, liposarcoma, glioblastoma, neuroblastoma, lymphoma, leukemia or melanoma.

9. The HDM201 for use according to claim 8 or the ABT-199 for use according to claim 8, wherein the cancer is colorectal cancer.

10. The HDM201 for use according to any one of claims 5 and 7 to 9 or the ABT-199 for use according to any one of claims 6 to 9, wherein the cancer comprises functional p53 or wild-type TP53.

11. The HDM201 for use according to any one of claims 5 and 7 to 10, or the ABT-199 for use according to any one of claims 6 to 10, wherein the cancer comprises one or more of KRAS mutation and/or BRAF mutation and/or MEK1 mutation and/or PIK3CA mutation and/or PIK3CA overexpression.

**Patentansprüche**

1. Pharmazeutische Kombination, umfassend den MDM2-Inhibitor (6S)-5-(5-Chlor-1-methyl-2-oxo-1,2-dihydropyridin-3-yl)-6-(4-chlorphenyl)-2-(2,4-dimethoxypyrimidin-5-yl)-1-(propan-2-yl)-5,6-dihydropyrrolo[3,4-d]imidazol-4(1H)-on (HDM201) oder ein pharmazeutisch unbedenkliches Salz davon und den Bc12-Inhibitor ABT-199 oder ein pharmazeutisch unbedenkliches Salz davon.

2. Pharmazeutische Kombination nach Anspruch 1, ferner umfassend einen MEK-Inhibitor aus der Gruppe bestehend aus Trametinib, 6-(4-Brom-2-fluorphenylamino)-7-fluor-3-methyl-3H-benzoimidazol-5-carbonsäure (2-hydroxyethoxy) amid, (S)-5-Fluor-2-(2-fluor-4-(methylthio)phenylamino)-N-(2-hydroxypropoxy)-1-methyl-6-oxo-1,6-dihydropyridin-3-carboxamid, PD0325901, PD-184352, RDEA119, XL518, AS-701255, AS-701173, AS703026, RDEA436, E6201, RO4987655, RG7167 und RG7420 oder ein pharmazeutisch unbedenkliches Salz davon.

3. Pharmazeutische Kombination nach einem der Ansprüche 1 bis 2, wobei die Kombination ferner einen EGFR-Inhibitor, einen PI3K-Inhibitor, einen BRAF-Inhibitor, einen CDK4/6-Inhibitor oder Paclitaxel umfasst.

4. Pharmazeutische Kombination nach Anspruch 1 oder Anspruch 2, wobei die Kombination ferner einen BRAF-Inhibitor und gegebenenfalls weiterhin einen cMET-Inhibitor umfasst.

5. HDM201 oder ein pharmazeutisch unbedenkliches Salz davon zur Verwendung bei der Behandlung von Krebs,

wobei die Behandlung ferner die Verabreichung von ABT-199 oder einem pharmazeutisch unbedenklichen Salz davon umfasst.

6. ABT-199 oder ein pharmazeutisch unbedenkliches Salz davon zur Verwendung bei der Behandlung von Krebs, wobei die Behandlung ferner die Verabreichung von HDM201 oder einem pharmazeutisch unbedenklichen Salz davon umfasst.

7. HDM201 zur Verwendung nach Anspruch 5 oder ABT-199 zur Verwendung nach Anspruch 6, wobei der Krebs aus der Gruppe bestehend aus einem benignen oder malignen Tumor der Lunge (einschließlich kleinzelligem Lungenkrebs und nichtkleinzelligem Lungenkrebs), Bronchuskrebs, Prostatakrebs, Brustkrebs (einschließlich sporadischer Brustkrebserkrankungen und Personen, die an Cowden-Krankheit leiden), Bauchspeicheldrüsenkrebs, Magen-Darm-Krebs, Kolonkrebs, Rektumkrebs, Kolonkarzinom, Kolorektalkrebs, Schilddrüsenkrebs, Leberkrebs, Gallengangkrebs, Krebs des intrahepatischen Gallengangs, Leberzellkrebs, Nebennierenkrebs, Magenkrebs, Darmkrebs, Gliom, Glioblastom, Endometriumkrebs, Nierenkrebs, Nierenbeckenkrebs, Blasenkrebs, Gebärmutterkrebs, Gebärmutterhalskrebs, Vaginalkrebs, Eierstockkrebs, multiplem Myelom, Speiseröhrenkrebs, Hals- oder Kopfkrebs, Gehirnkrebs, Mundhöhlen- und Rachenkrebs, Kehlkopfkrebs, Dünndarmskrebs, einem Melanom, villösem Kolonadenom, einem Sarkom, einer Neoplasie, einer Neoplasie mit epithelialem Charakter, einem Mammakarzinom, Basalzellkarzinom, Plattenepithelkarzinom, aktinischer Keratose, Polycythemia vera, essenzieller Thrombozythämie, einer Leukämie (einschließlich akuter myeloischer Leukämie, chronischer myeloischer Leukämie, lymphozytischer Leukämie und myeloischer Leukämie), einem Lymphom (einschließlich Non-Hodgkin-Lymphom und Hodgkin-Lymphom), Myelofibrose mit myeloischer Metaplasie, Morbus Waldenström und Barret-Adenokarzinom ausgewählt ist.

8. HDM201 zur Verwendung nach Anspruch 7 oder ABT-199 zur Verwendung nach Anspruch 7, wobei es sich bei dem Krebs um Kolorektalkrebs, Liposarkom, Glioblastom, Neuroblastom, Lymphom, Leukämie oder Melanom handelt.

9. HDM201 zur Verwendung nach Anspruch 8 oder ABT-199 zur Verwendung nach Anspruch 8, wobei es sich bei dem Krebs um Kolorektalkrebs handelt.

10. HDM201 zur Verwendung nach einem der Ansprüche 5 und 7 bis 9 oder ABT-199 zur Verwendung nach einem der Ansprüche 6 bis 9, wobei der Krebs funktionelles p53 umfasst oder vom TP53-Wildtyp ist.

11. HDM201 zur Verwendung nach einem der Ansprüche 5 und 7 bis 10 oder ABT-199 zur Verwendung nach einem der Ansprüche 6 bis 10, wobei der Krebs eine oder mehrere KRAS-Mutationen und/oder BRAF-Mutationen und/oder MEK1-Mutationen und/oder PIK3CA-Mutationen und/oder PIK3CA-Überexpression umfasst.

## Revendications

1. Combinaison pharmaceutique comprenant l'inhibiteur de MDM2 (6S)-5-(5-chloro-1-méthyl-2-oxo-1,2-dihydropyridin-3-yl)-6-(4-chlorophényl)-2-(2,4-diméthoxypyrimidin-5-yl)-1-(propan-2-yl)-5,6-dihydropyrrolo[3,4-d]imidazol-4(1H)-one (HDM201), ou un sel pharmaceutiquement acceptable correspondant, et l'inhibiteur de Bcl2 : ABT-199, ou un sel pharmaceutiquement acceptable correspondant.

2. Combinaison pharmaceutique selon la revendication 1 comprenant en outre un inhibiteur de MEK choisi dans le groupe constitué par tramétinib, acide 6-(4-bromo-2-fluorophénylamino)-7-fluoro-3-méthyl-3H-benzoimidazole-5-carboxylique (2-hydroxyéthoxy)-amide, (S)-5-fluoro-2-(2-fluoro-4-(méthylthio)phénylamino)-N-(2-hydroxypropoxy)-1-méthyl-6-oxo-1,6-dihydropyridine-3-carboxamide, PD0325901, PD-184352, RDEA119, XL518, AS-701255, AS-701173, AS703026, RDEA436, E6201, RO4987655, RG7167, et RG7420 ou un sel pharmaceutiquement acceptable correspondant.

3. Combinaison pharmaceutique selon l'une quelconque des revendications 1 à 2, la combinaison comprenant en outre un inhibiteur d'EGFR, un inhibiteur de PI3K, un inhibiteur de BRAF, un inhibiteur de CDK4/6 ou le paclitaxel.

4. Combinaison pharmaceutique selon la revendication 1 ou la revendication 2, la combinaison comprenant en outre un inhibiteur de BRAF, et éventuellement comprenant encore en outre un inhibiteur de cMET.

5. HDM201, ou sel pharmaceutiquement acceptable correspondant pour une utilisation dans le traitement d'un cancer,

le traitement comprenant en outre une administration d'ABT-199, ou d'un sel pharmaceutiquement acceptable correspondant.

6. ABT-199 ou sel pharmaceutiquement acceptable correspondant pour une utilisation dans le traitement d'un cancer, le traitement comprenant en outre une administration d'HDM201, ou d'un sel pharmaceutiquement acceptable correspondant.

7. HDM201 pour une utilisation selon la revendication 5 ou ABT-199 pour une utilisation selon la revendication 6, le cancer étant choisi dans le groupe constitué par une tumeur bénigne ou maligne du poumon (y compris le cancer du poumon à petites cellules et le cancer du poumon non à petites cellules), un cancer des bronches, de la prostate, du sein (y compris les cancers du sein sporadiques et les personnes souffrant de la maladie de Cowden), du pancréas, du tractus gastro-intestinal, du côlon, du rectum, un carcinome du côlon, un cancer colorectal, de la thyroïde, du foie, des voies biliaires, du canal biliaire intrahépatique, hépatocellulaire, de la glande surrénale, de l'estomac, gastrique, un gliome, un glioblastome, un cancer de l'endomètre, du rein, du bassinet du rein, de la vessie, de l'utérus, du col de l'utérus, du vagin, de l'ovaire, un myélome multiple, un cancer de l'œsophage, du cou ou de la tête, du cerveau, de la cavité buccale et du pharynx, du larynx, de l'intestin grêle, un mélanome, un adénome villositaire du côlon, un sarcome, une néoplasie, une néoplasie à caractère épithélial, un carcinome mammaire, un carcinome basocellulaire, un carcinome spinocellulaire, une kératose actinique, une polyglobulie, une thrombocytémie essentielle, une leucémie (y compris la leucémie myélogène aiguë, la leucémie myélogène chronique, la leucémie lymphocytaire et la leucémie myéloïde), un lymphome (y compris le lymphome non hodgkinien et le lymphome de Hodgkin), une myélofibrose avec métaplasie myéloïde, la maladie de Waldenstroem et un adénocarcinome de Barret.

8. HDM201 pour une utilisation selon la revendication 7 ou ABT-199 pour une utilisation selon la revendication 7, le cancer étant un cancer colorectal, un liposarcome, un glioblastome, un neuroblastome, un lymphome, une leucémie ou un mélanome.

9. HDM201 pour une utilisation selon la revendication 8 ou ABT-199 pour une utilisation selon la revendication 8, le cancer étant un cancer colorectal.

10. HDM201 pour une utilisation selon l'une quelconque des revendications 5 et 7 à 9, ou ABT-199 pour une utilisation selon l'une quelconque des revendications 6 à 9, le cancer comprenant p53 fonctionnel ou TP53 de type sauvage.

11. HDM201 pour une utilisation selon l'une quelconque des revendications 5 et 7 à 10, ou ABT-199 pour une utilisation selon l'une quelconque des revendications 6 à 10, le cancer comprenant l'une ou plusieurs parmi une mutation KRAS et/ou une mutation BRAF et/ou une mutation MEK1 et/ou une mutation PIK3CA et/ou une surexpression de PIK3CA.

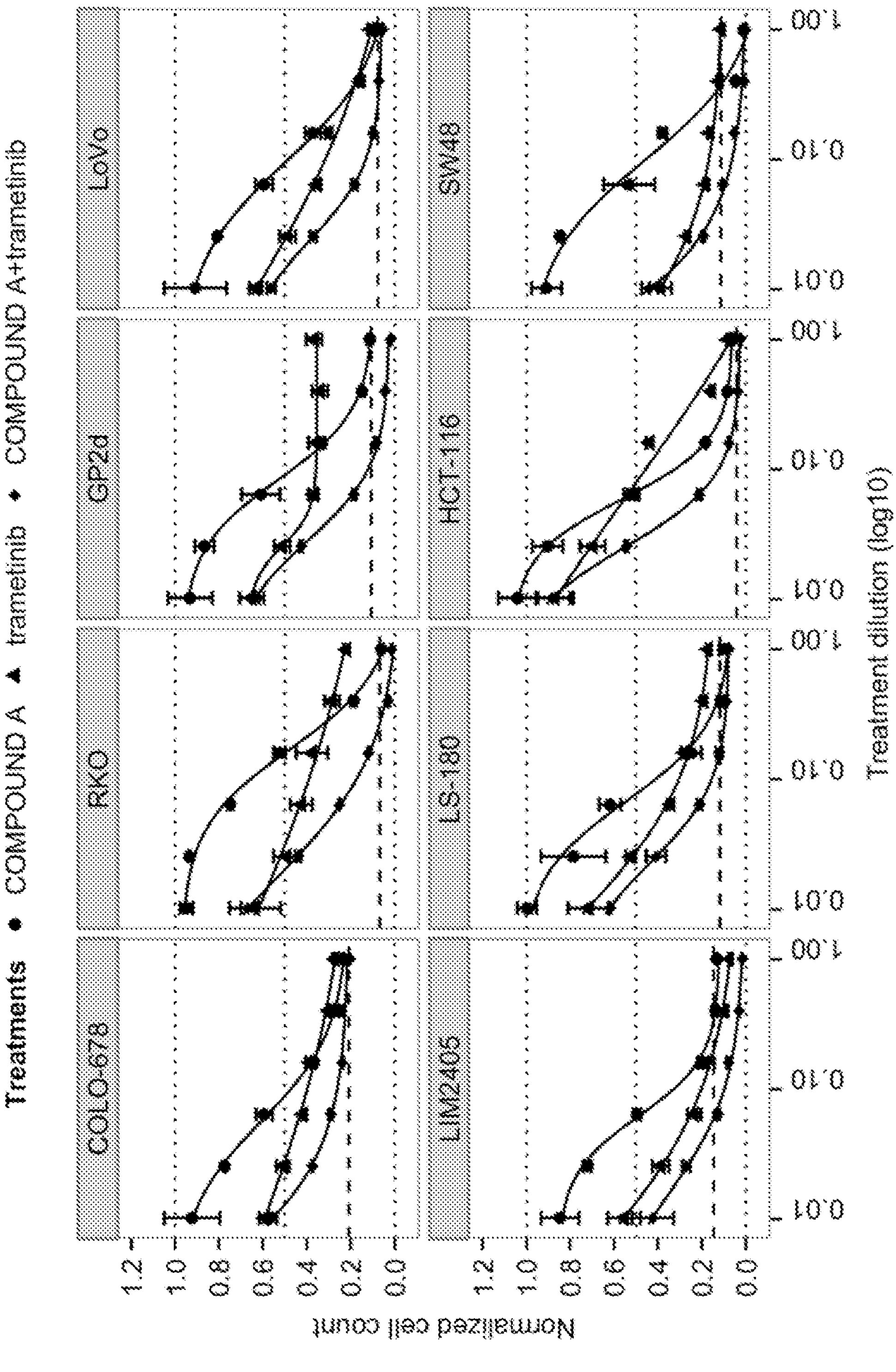
Treatments
COMPOUND A
trametinib
COMPOUND A+trametinib
COLO-678
RKO
GP2d
LoVo
LIM2405
LS-180
HCT-116
SW48
Normalized cell count
Treatment dilution (log10)

FIG. 1

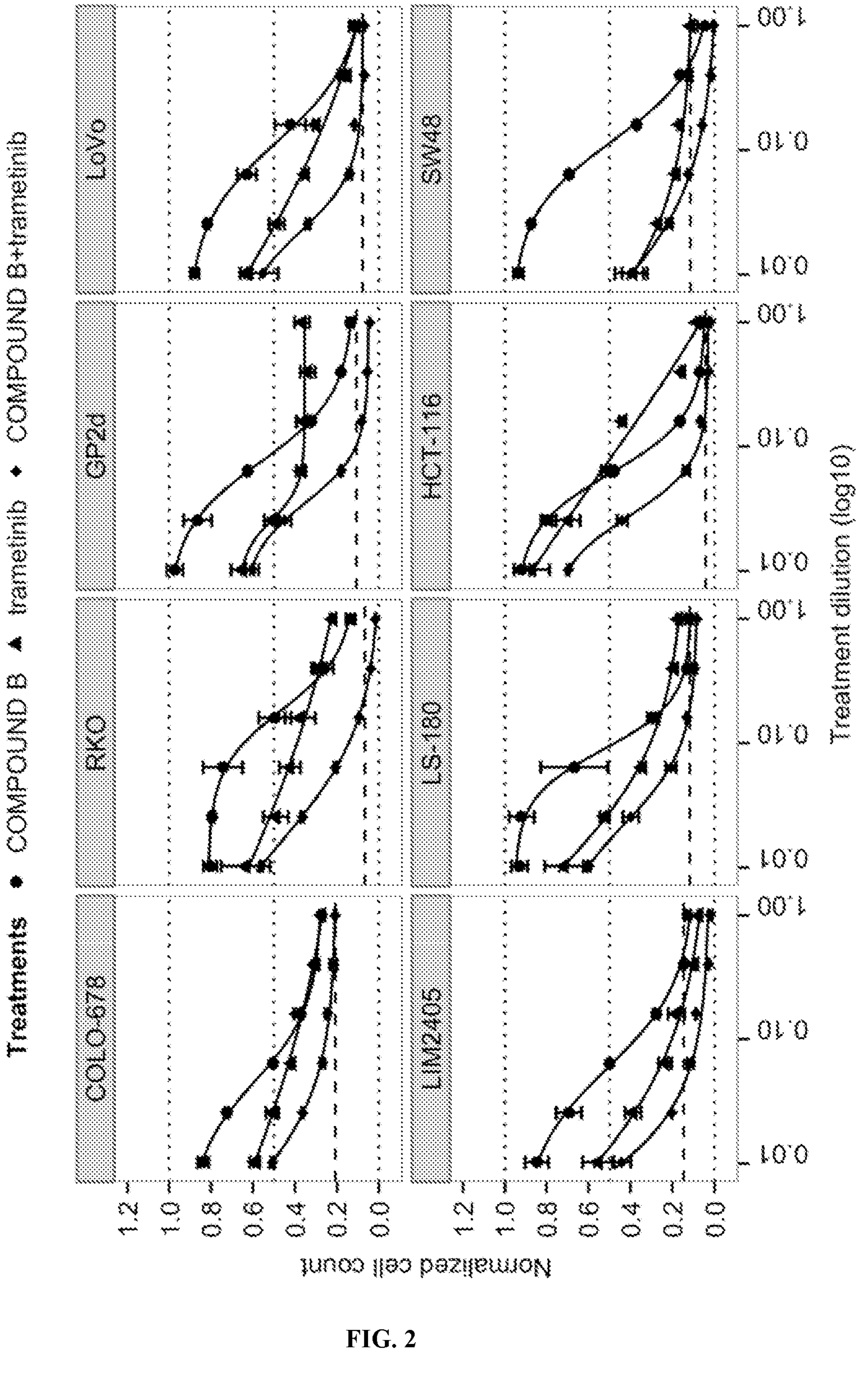
Treatments
COMPOUND B
trametinib
COMPOUND B+trametinib
COLO-678
RKO
GP2d
LoVo
LIM2405
LS-180
HCT-116
SW48
Normalized cell count
1.2
1.0
0.8
0.6
0.4
0.2
0.0
0.01
0.10
1.00
Treatment dilution (log10)

FIG. 2

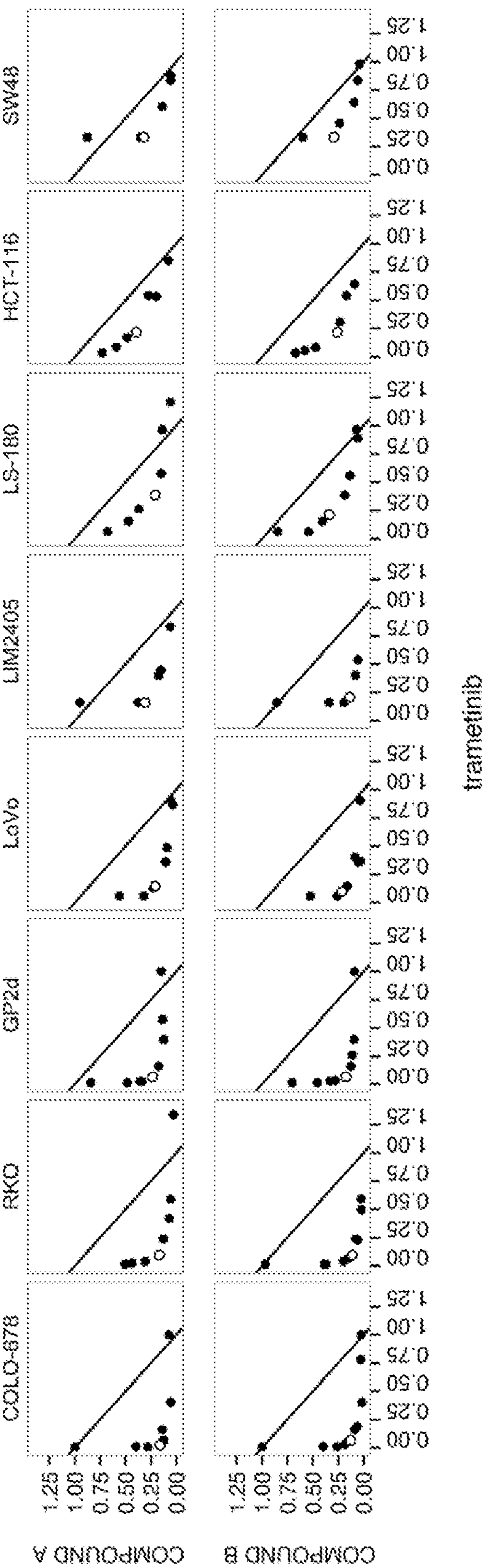
COLO-678
RKO
GP2d
LoVo
LIM2405
LS-180
HCT-116
SW48
COMPOUND A
COMPOUND B
trametinib
0.00
0.25
0.50
0.75
1.00
1.25

FIG. 3

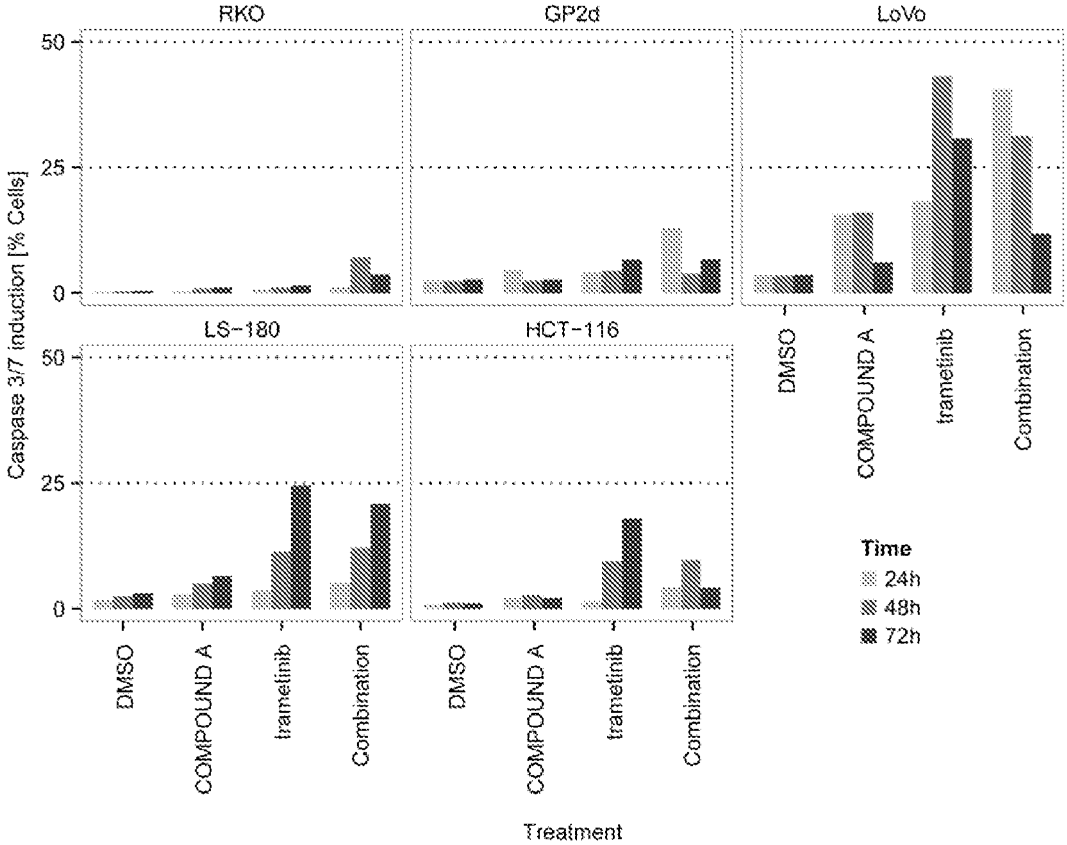
RKO
GP2d
LoVo
LS−180
HCT−116
Caspase 3/7 induction [% Cells]
50
25
0
DMSO
COMPOUND A
trametinib
Combination
Treatment
Time
24h
48h
72h

FIG. 4

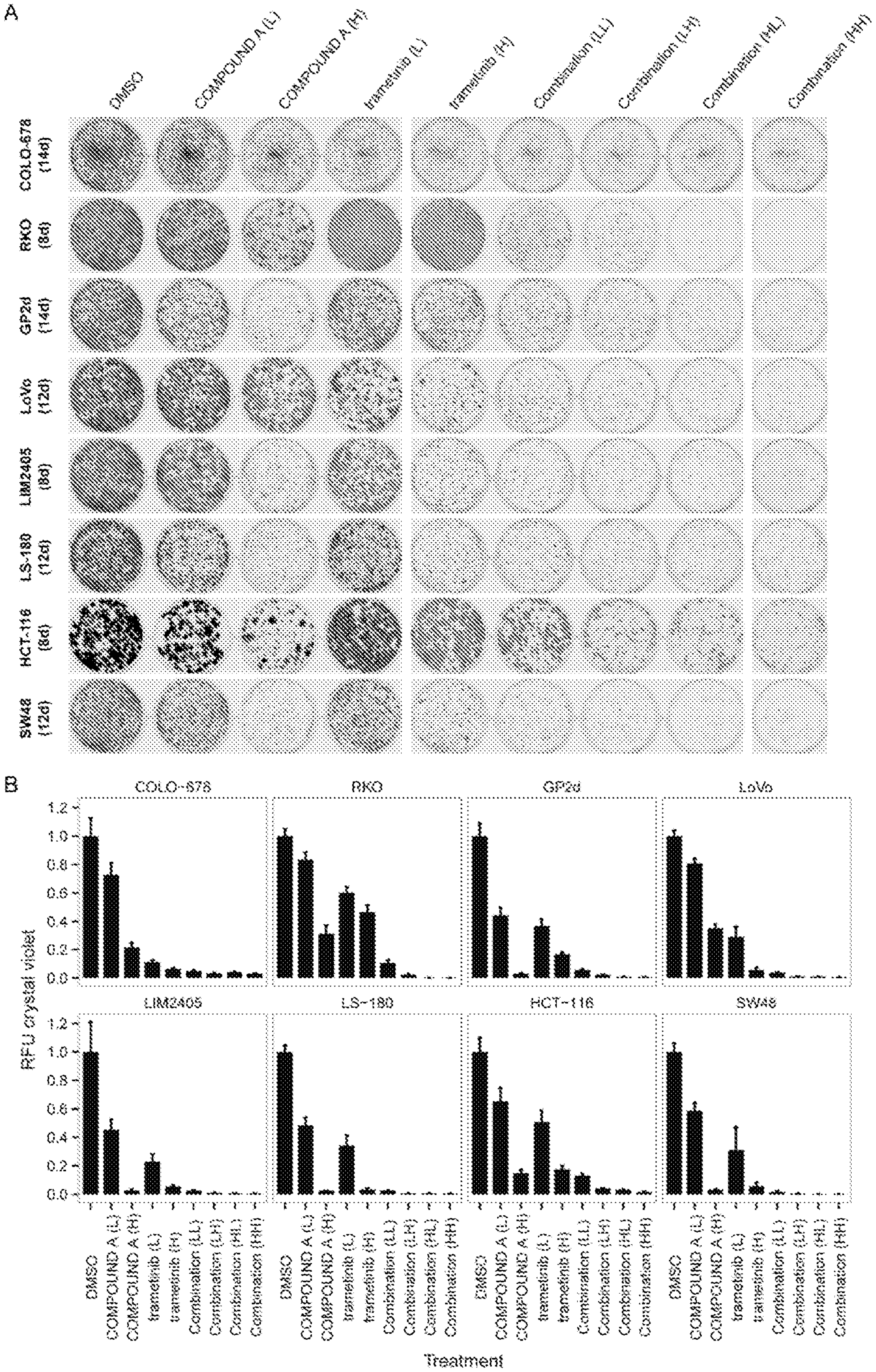
A
DMSO
COMPOUND A (L)
COMPOUND A (H)
trametinib (L)
trametinib (H)
Combination (LL)
Combination (LH)
Combination (HL)
Combination (HH)
COLO-678 (14d)
RKO (8d)
GP2d (14d)
LoVo (12d)
LIM2405 (8d)
LS-180 (12d)
HCT-116 (8d)
SW48 (12d)
B
COLO-678
RKO
GP2d
LoVo
LIM2405
LS-180
HCT-116
SW48
RFU crystal violet
Treatment

FIG. 5

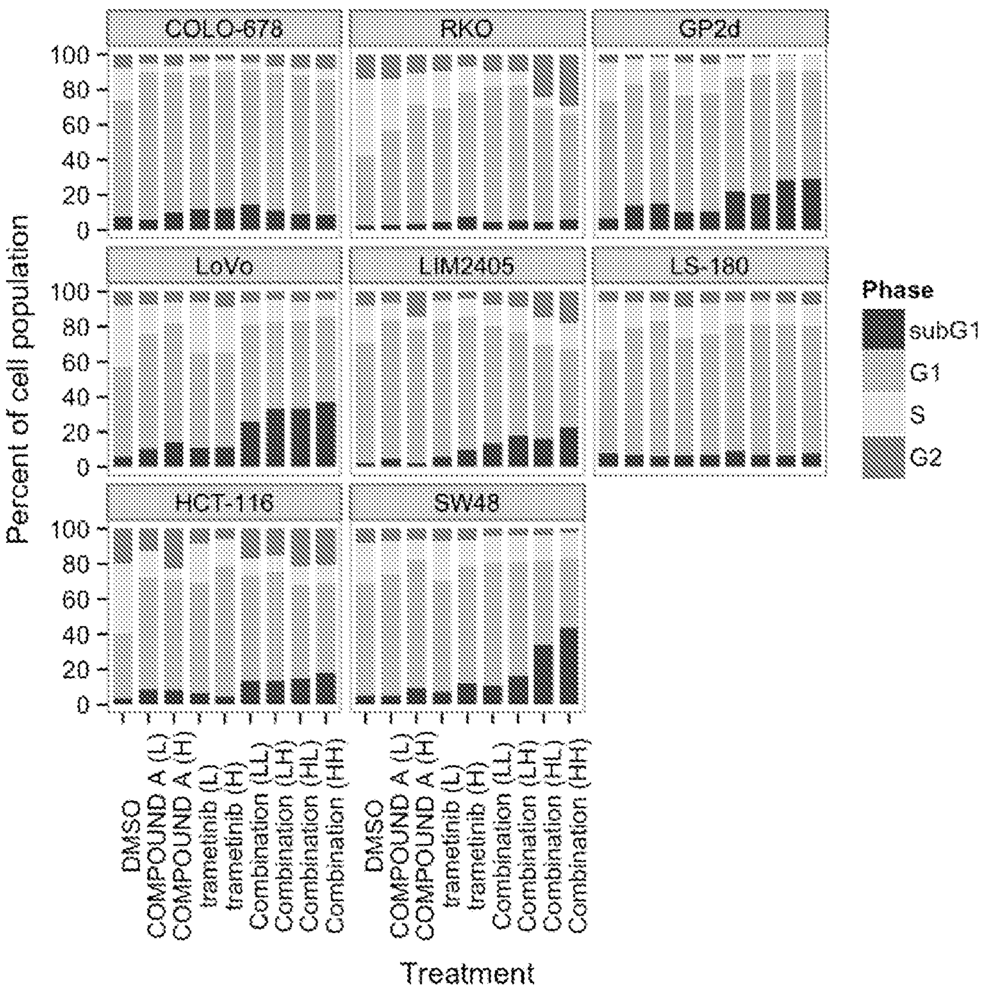
COLO-678
RKO
GP2d
LoVo
LIM2405
LS-180
HCT-116
SW48
Percent of cell population
0
20
40
60
80
100
Phase
subG1
G1
S
G2
DMSO
COMPOUND A (L)
COMPOUND A (H)
trametinib (L)
trametinib (H)
Combination (LL)
Combination (LH)
Combination (HL)
Combination (HH)
Treatment

FIG. 6

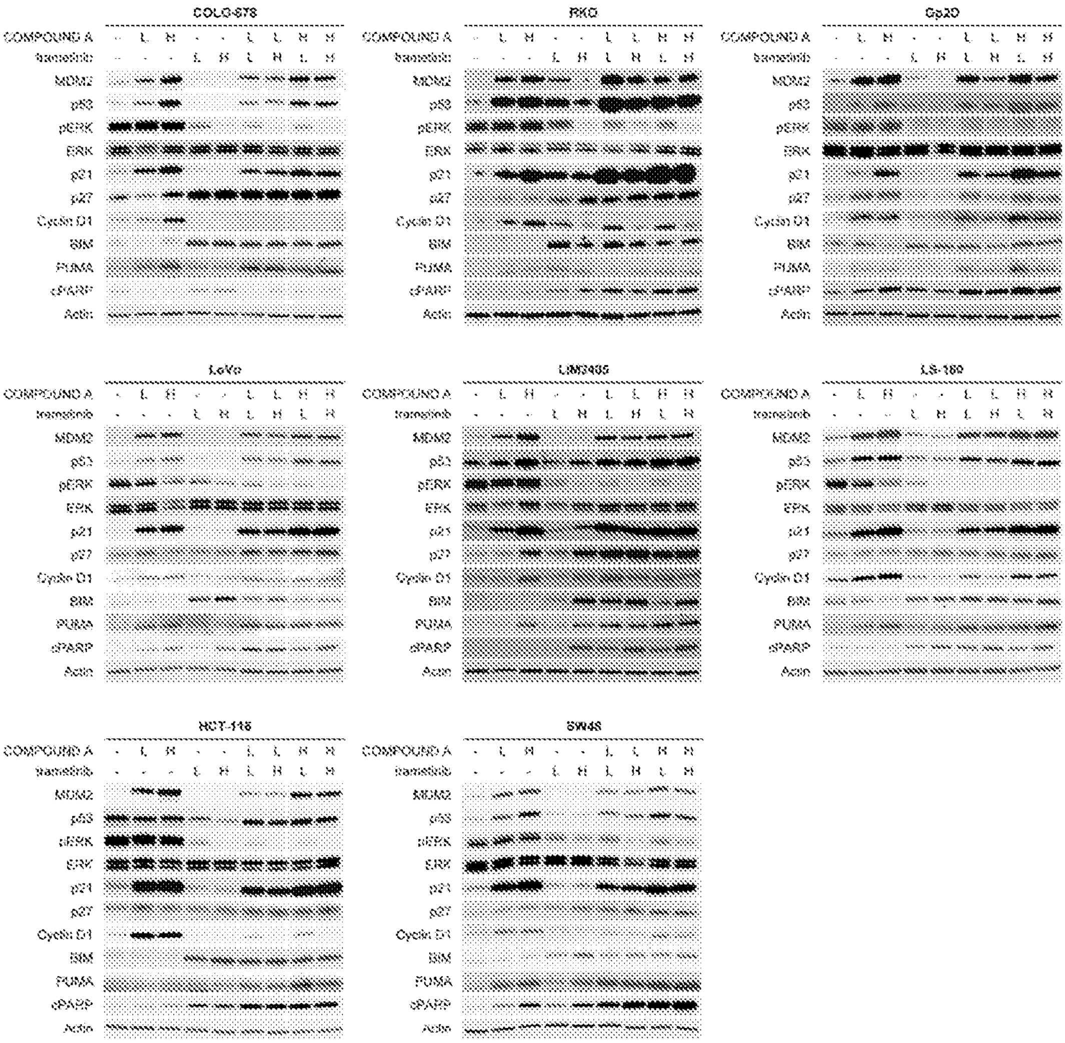

FIG. 7

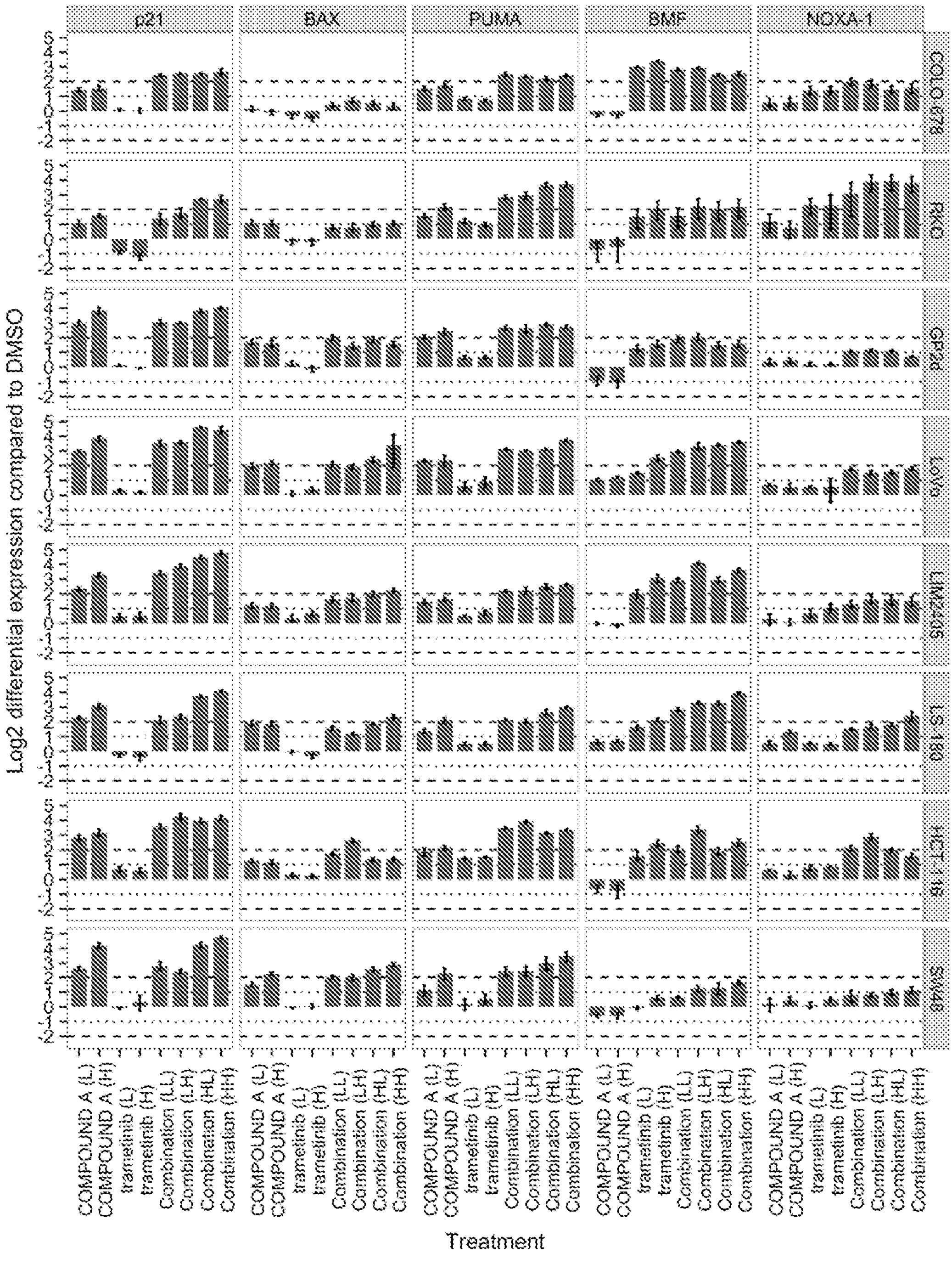
p21
BAX
PUMA
BMF
NOXA-1
COLO-678
RKO
GP2d
LoVo
LIM2405
LS-180
HCT-116
SW48
Log2 differential expression compared to DMSO
COMPOUND A (L)
COMPOUND A (H)
trametinib (L)
trametinib (H)
Combination (LL)
Combination (LH)
Combination (HL)
Combination (HH)
Treatment

FIG. 8

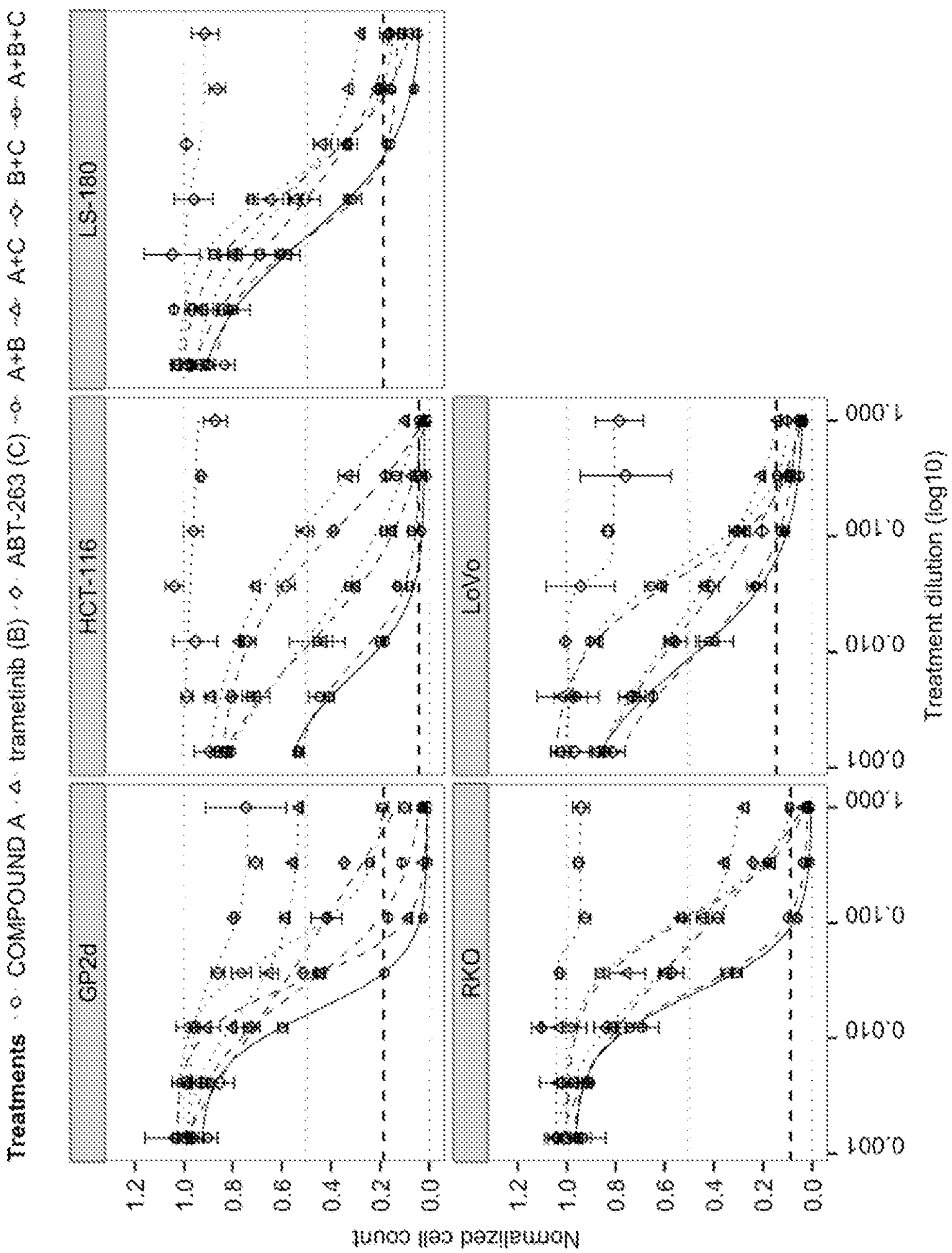
Treatments
COMPOUND A
trametinib (B)
ABT-263 (C)
A+B
A+C
B+C
A+B+C
GP2d
HCT-116
LS-180
RKO
LoVo
Normalized cell count
1.2
1.0
0.8
0.6
0.4
0.2
0.0
0.001
0.010
0.100
1.000
Treatment dilution (log10)

FIG. 9

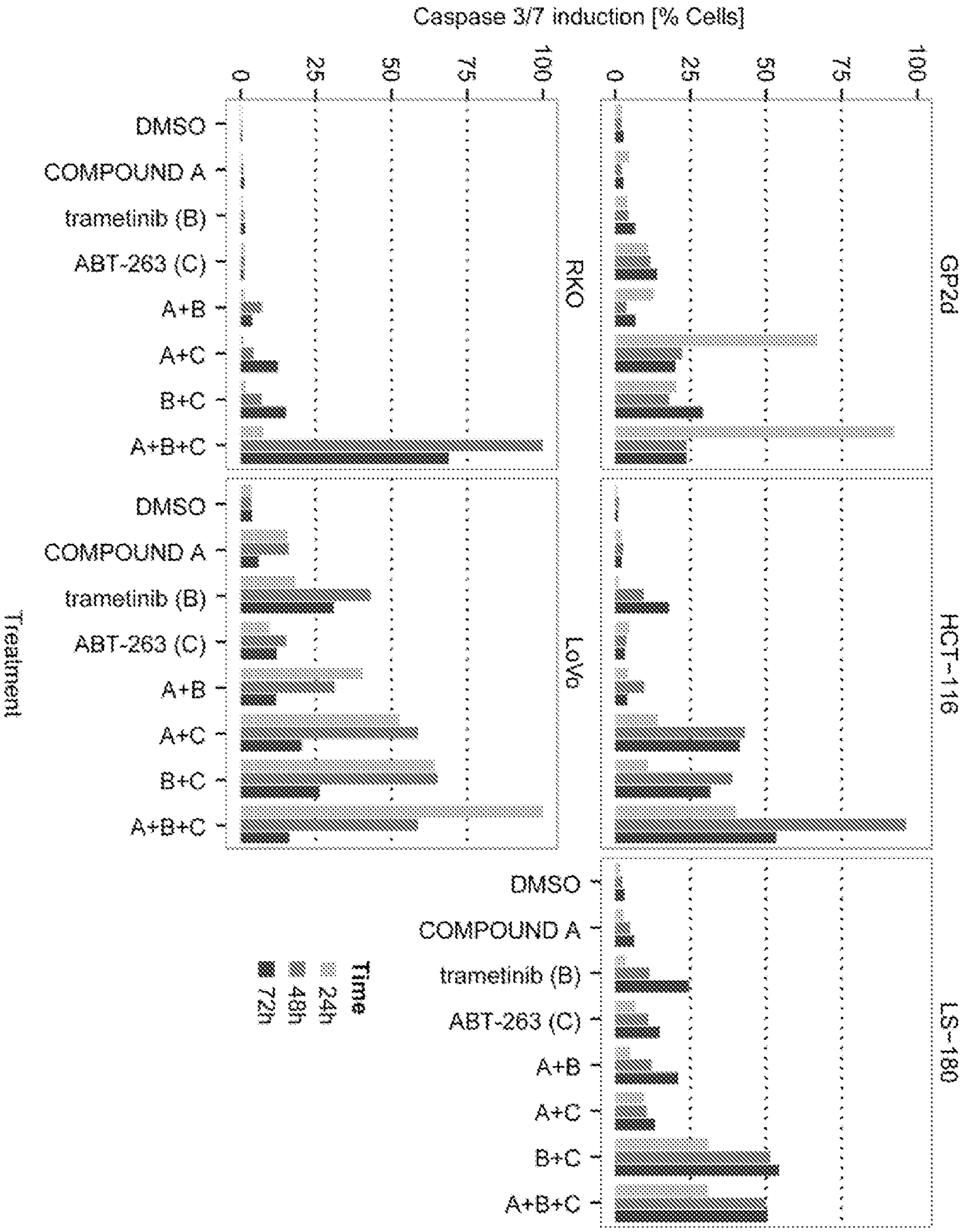
Caspase 3/7 induction [% Cells]
0
25
50
75
100
GP2d
HCT−116
LS−180
RKO
LoVo
DMSO
COMPOUND A
trametinib (B)
ABT-263 (C)
A+B
A+C
B+C
A+B+C
Treatment
Time
24h
48h
72h

**FIG. 10**

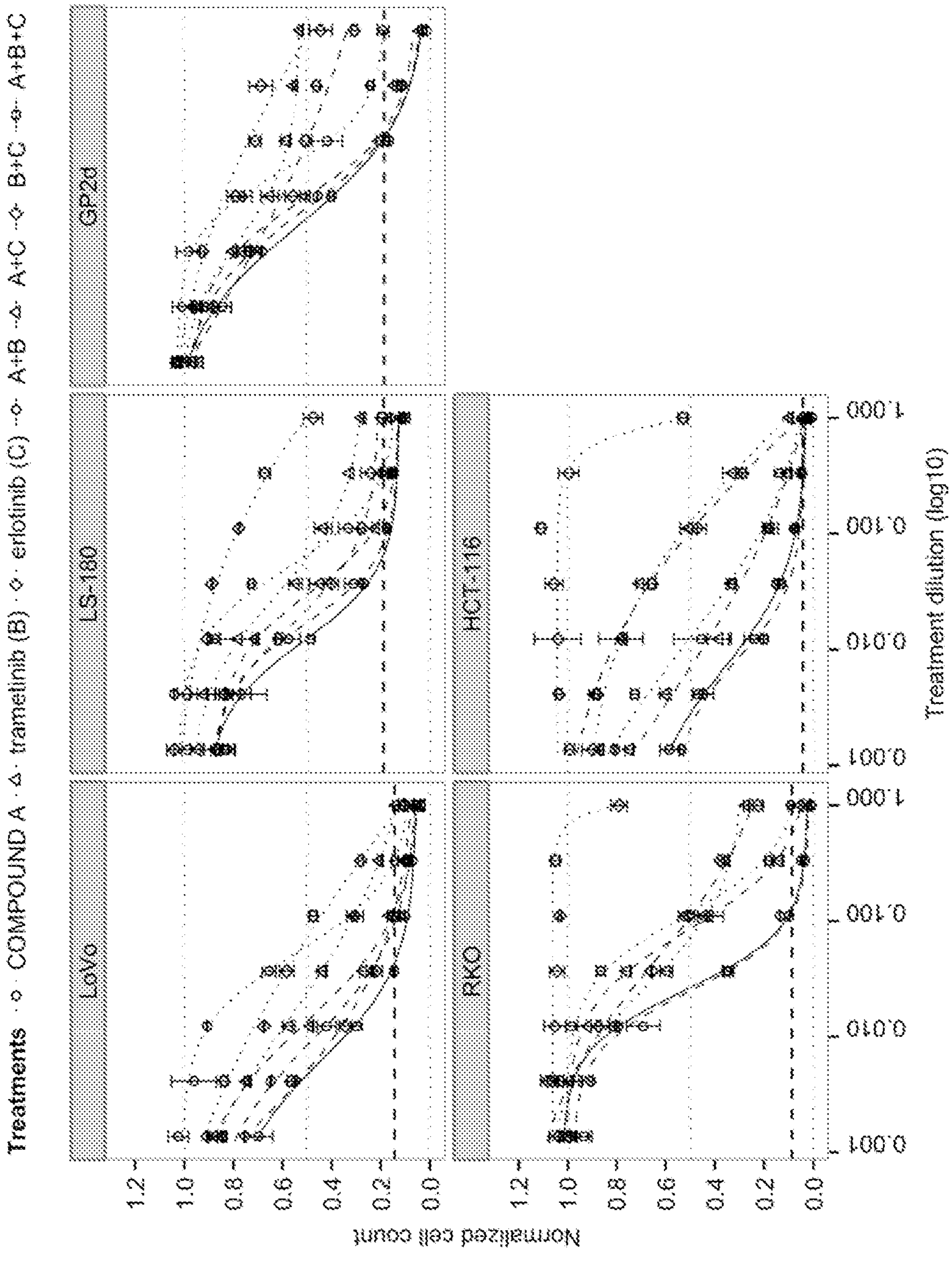
Treatments
COMPOUND A
trametinib (B)
erlotinib (C)
A+B
A+C
B+C
A+B+C
LoVo
LS-180
GP2d
RKO
HCT-116
Normalized cell count
Treatment dilution (log10)

FIG. 11

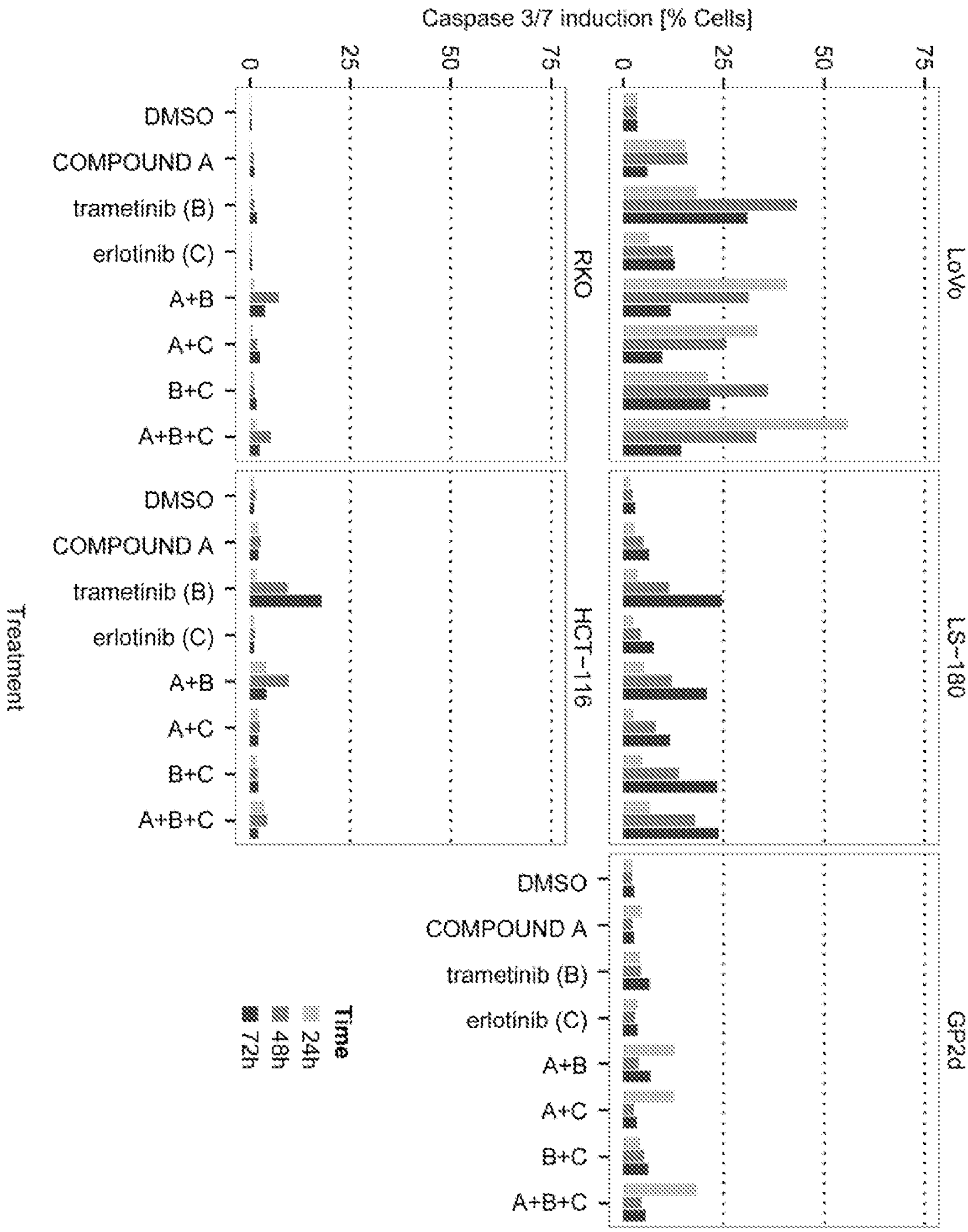
Caspase 3/7 induction [% Cells]
0
25
50
75
LoVo
LS−180
GP2d
RKO
HCT−116
DMSO
COMPOUND A
trametinib (B)
erlotinib (C)
A+B
A+C
B+C
A+B+C
Treatment
Time
24h
48h
72h

FIG. 12

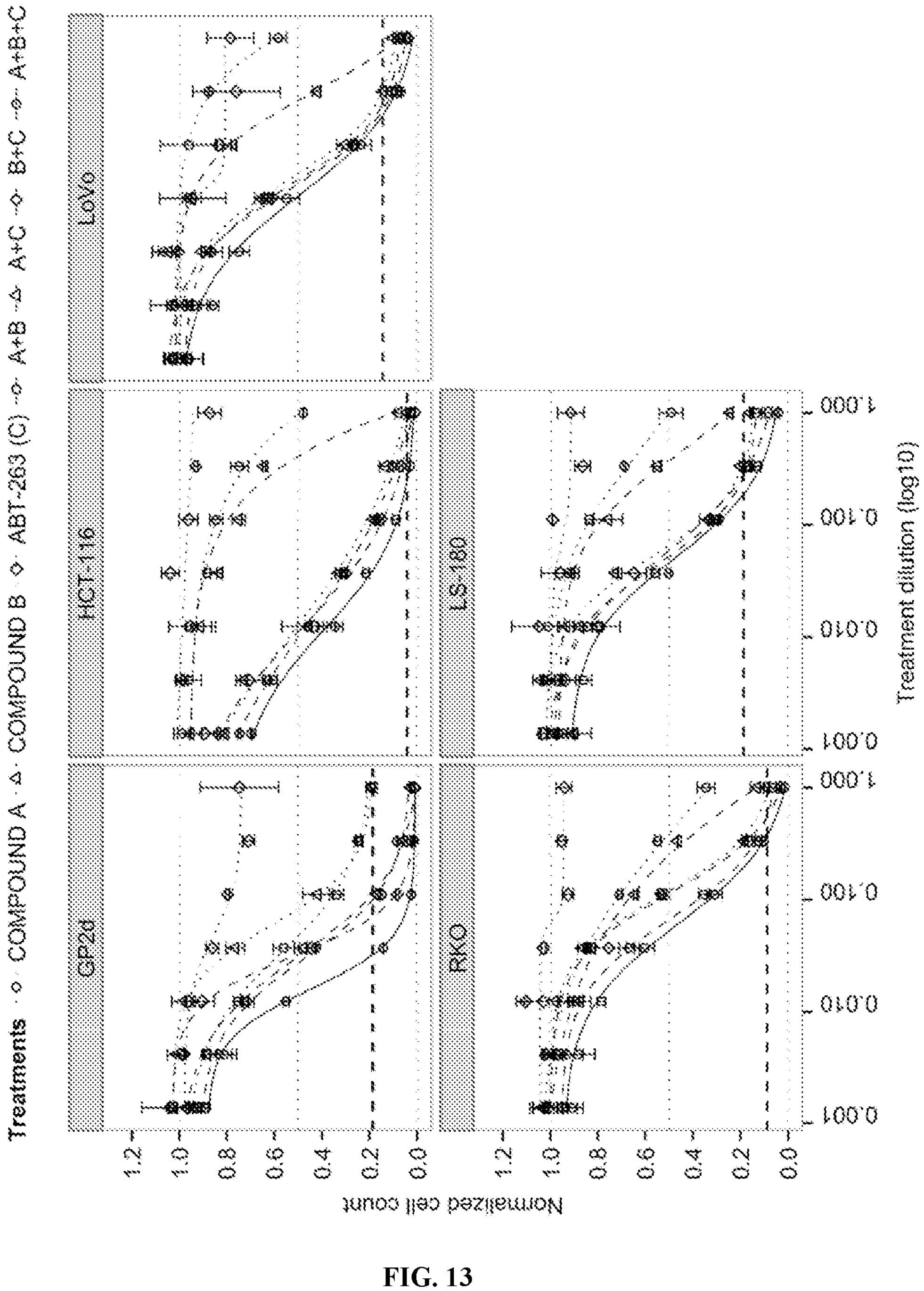
Treatments
COMPOUND A
COMPOUND B
ABT-263 (C)
A+B
A+C
B+C
A+B+C
GP2d
HCT-116
LoVo
RKO
LS-180
Normalized cell count
Treatment dilution (log10)
0.0
0.2
0.4
0.6
0.8
1.0
1.2
0.001
0.010
0.100
1.000

FIG. 13

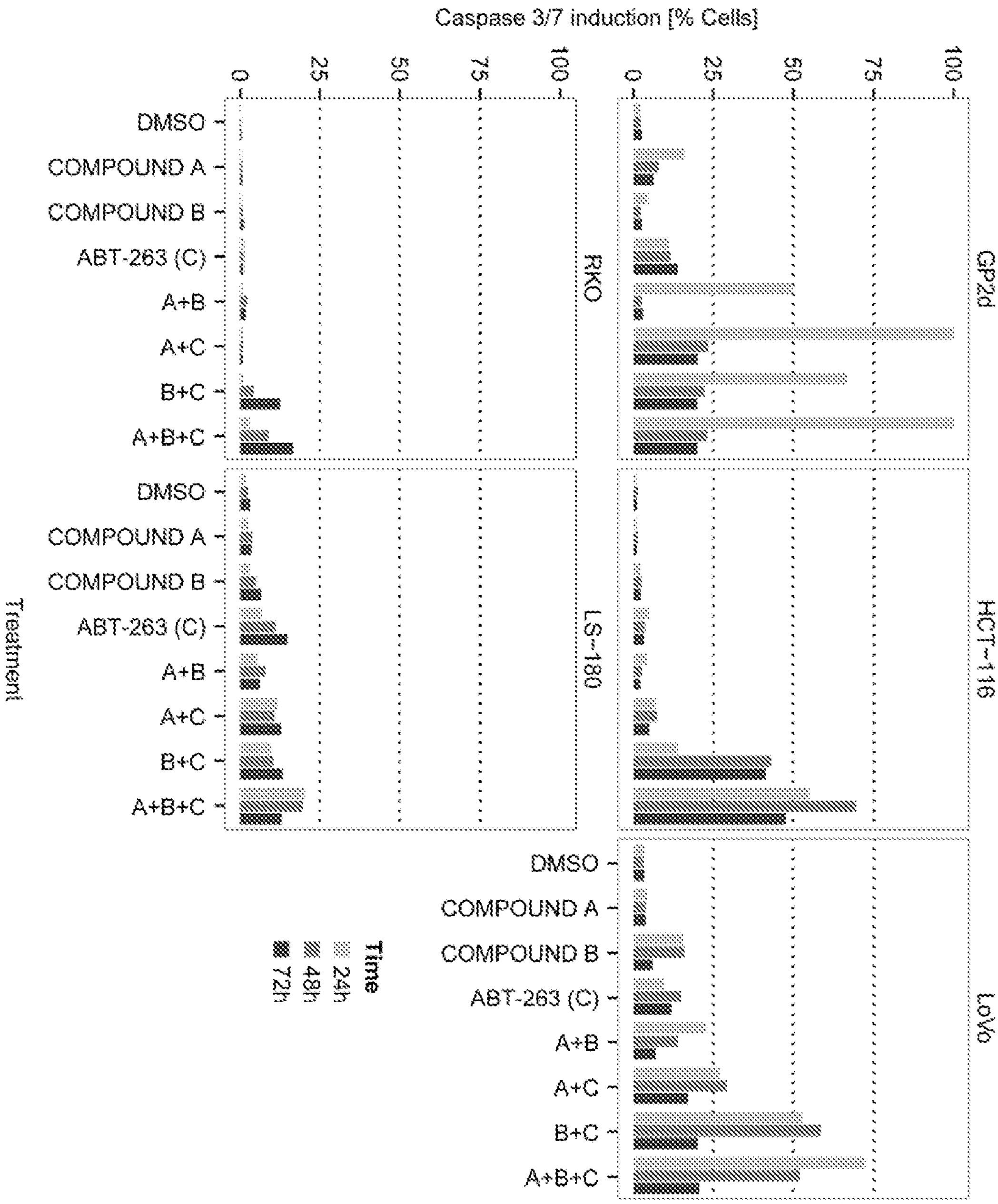
Caspase 3/7 induction [% Cells]
0
25
50
75
100
GP2d
HCT-116
LoVo
RKO
LS-180
DMSO
COMPOUND A
COMPOUND B
ABT-263 (C)
A+B
A+C
B+C
A+B+C
Treatment
Time
24h
48h
72h

FIG. 14

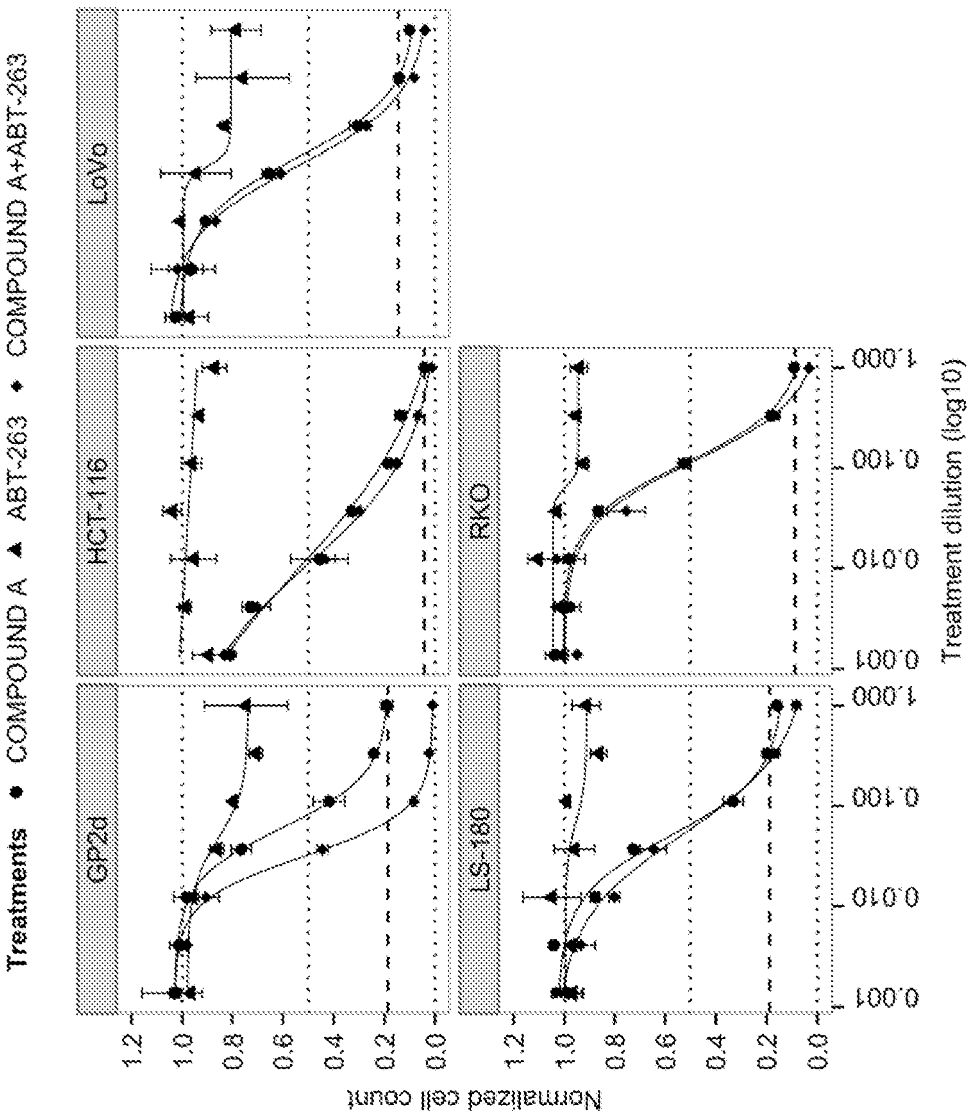
Treatments
COMPOUND A
ABT-263
COMPOUND A+ABT-263
GP2d
HCT-116
LoVo
LS-180
RKO
Normalized cell count
Treatment dilution (log10)
0.001
0.010
0.100
1.000
0.0
0.2
0.4
0.6
0.8
1.0
1.2

**FIG. 15**

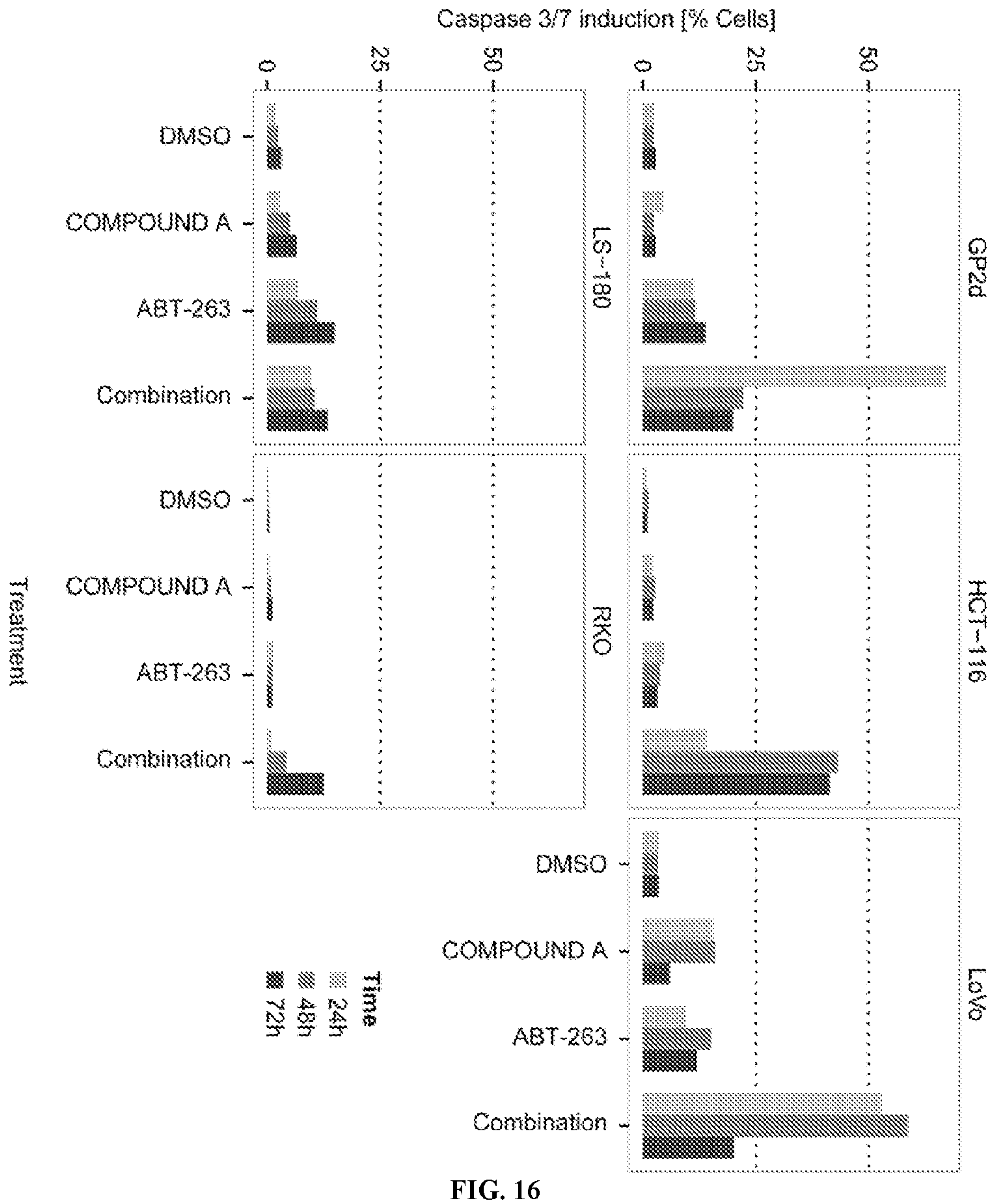
Caspase 3/7 induction [% Cells]
0
25
50
GP2d
HCT−116
LoVo
LS−180
RKO
DMSO
COMPOUND A
ABT-263
Combination
Treatment
Time
24h
48h
72h

FIG. 16

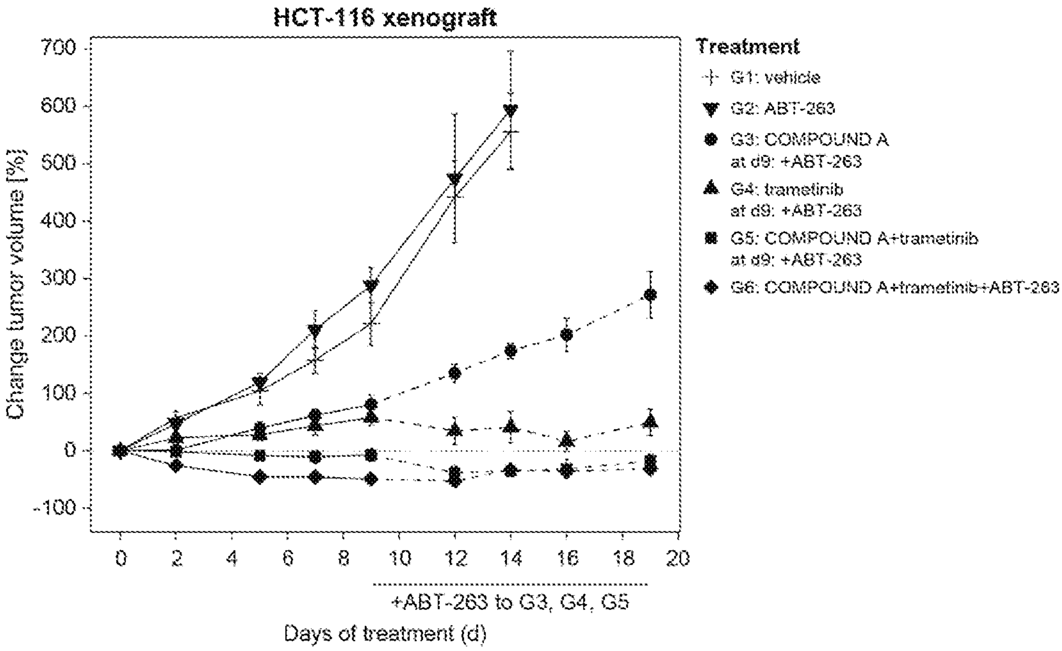
HCT-116 xenograft
Change tumor volume [%]
700
600
500
400
300
200
100
0
-100
0
2
4
6
8
10
12
14
16
18
20
+ABT-263 to G3, G4, G5
Days of treatment (d)
Treatment
G1: vehicle
G2: ABT-263
G3: COMPOUND A
at d9: +ABT-263
G4: trametinib
at d9: +ABT-263
G5: COMPOUND A+trametinib
at d9: +ABT-263
G6: COMPOUND A+trametinib+ABT-263

FIG. 17

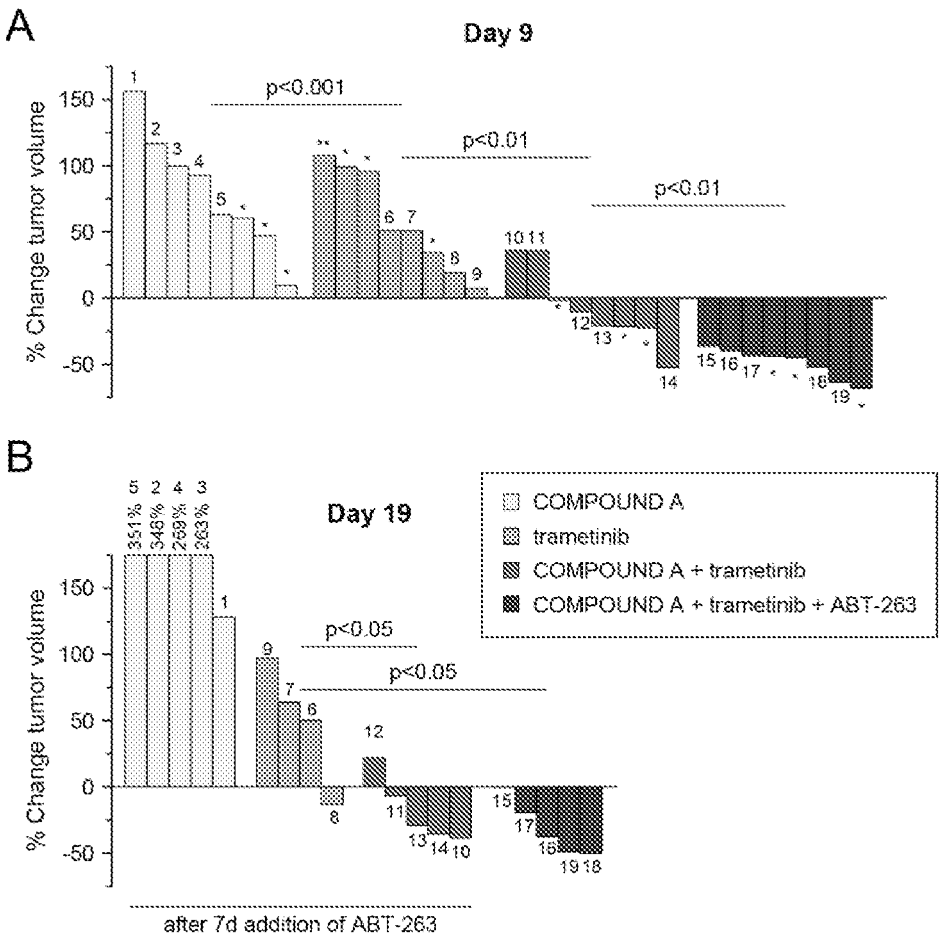
A
Day 9
% Change tumor volume
p<0.001
p<0.01
p<0.01
B
Day 19
351% 346% 269% 263%
p<0.05
p<0.05
after 7d addition of ABT-263
COMPOUND A
trametinib
COMPOUND A + trametinib
COMPOUND A + trametinib + ABT-263

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013111105 A
- JP 74057 T
- WO 05121142 A
- WO 03077914 A
- WO 2007044084 A
- WO 0206213 A
- WO 2006122806 A
- WO 07084786 A
- WO 2007084786 A
- WO 2010029082 A


### Non-patent literature cited in the description


- **K KOJIMA et al.** Cell cycle. *Landes Bioscience,* 01 December 2006, vol. 5 (23), 2778-2786
- **HOLFORD, N. H. G. ; SCHEINER, L. B.** *Clin. Pharmacokinet.,* 1981, vol. 6, 429-453
- **LOEWE, S. ; MUISCHNEK, H.** *Arch. Exp. Pathol Pharmacol.,* 1926, vol. 114, 313-326
- **CHOU, T. C. ; TALALAY, P.** *Adv. Enzyme Regul.,* 1984, vol. 22, 27-55
- **GOTO et al.** *Journal of Pharmacology and Experimental Therapeutics,* 2009, vol. 3331 (2), 485-495
- Remington's Pharmaceutical Sciences. Mack Printing Company, 1990, 1289-1329
- The Handbook of Pharmaceutical Excipients. American Pharmaceuticals Association, 2003
- **REMINGTON.** Science and Practice of Pharmacy. Lippincott Williams & Wilkins, 2003
- **ARRINGTON, A. K. ; E. L. HEINRICH ; W. LEE ; M. DULDULAO ; S. PATEL ; J. SANCHEZ ; J. GARCIA-AGUILAR ; J. KIM.** Prognostic and predictive roles of KRAS mutation in colorectal cancer. *Int J Mol Sci,* 2012, vol. 13 (10), 12153-12168
- **BERENBAUM, M. C.** What is synergy?. *Pharmacol Rev,* 1989, vol. 41 (2), 93-141
- **BOZIC, I. ; J. G. REITER ; B. ALLEN ; T. ANTAL ; K. CHATTERJEE ; P. SHAH ; Y. S. MOON ; A. YAQUBIE ; N. KELLY ; D. T. LE.** Evolutionary dynamics of cancer in response to targeted combination therapy. *Elife,* 2013, vol. 2, e00747
- **BRANA, I. ; L. L. SIU.** Clinical development of phosphatidylinositol 3-kinase inhibitors for cancer treatment. *BMC Med,* 2012, vol. 10, 161
- **CANCER GENOME ATLAS, N.** Comprehensive molecular characterization of human colon and rectal cancer. *Nature,* 2012, vol. 487 (7407), 330-337
- **CHANDARLAPATY, S.** Negative feedback and adaptive resistance to the targeted therapy of cancer. *Cancer Discov,* 2012, vol. 2 (4), 311-319
- **CHAPMAN, P. B. ; D. B. SOLIT ; N. ROSEN.** Combination of RAF and MEK inhibition for the treatment of BRAF-mutated melanoma: feedback is not encouraged. *Cancer Cell,* 2014, vol. 26 (5), 603-604
- **CHATTERJEE, M. S. ; J. E. PURVIS ; L. F. BRASS ; S. L. DIAMOND.** Pairwise agonist scanning predicts cellular signaling responses to combinatorial stimuli. *Nat Biotechnol,* 2010, vol. 28 (7), 727-732
- **CHOU, T. C. ; P. TALALAY.** Generalized equations for the analysis of inhibitions of Michaelis-Menten and higher-order kinetic systems with two or more mutually exclusive and nonexclusive inhibitors. *Eur J Biochem,* 1981, vol. 115 (1), 207-216
- **DEVITA, V. T., JR.** Single agent versus combination chemotherapy. *CA Cancer J Clin,* 1975, vol. 25 (3), 152-158
- **DOEBELE, R. C. ; A. B. PILLING ; D. L. AISNER ; T. G. KUTATELADZE ; A. T. LE ; A. J. WEICKHARDT ; K. L. KONDO ; D. J. LINDERMAN ; L. E. HEASLEY ; W. A. FRANKLIN.** Mechanisms of resistance to crizotinib in patients with ALK gene rearranged non-small cell lung cancer. *Clin Cancer Res,* 2012, vol. 18 (5), 1472-1482
- **DRUKER, B. J.** Translation of the Philadelphia chromosome into therapy for CML. *Blood,* 2008, vol. 112 (13), 4808-4817
- **DUNCAN, J. S. ; M. C. WHITTLE ; K. NAKAMURA ; A. N. ABELL ; A. A. MIDLAND ; J. S. ZAWISTOWSKI ; N. L. JOHNSON ; D. A. GRANGER ; N. V. JORDAN ; D. B. DARR.** Dynamic reprogramming of the kinome in response to targeted MEK inhibition in triple-negative breast cancer. *Cell,* 2012, vol. 149 (2), 307-321
- **FABER, A. C. ; E. M. COFFEE ; C. COSTA ; A. DASTUR ; H. EBI ; A. N. HATA ; A. T. YEO ; E. J. EDELMAN ; Y. SONG ; A. T. TAM.** mTOR inhibition specifically sensitizes colorectal cancers with KRAS or BRAF mutations to BCL-2/BCL-XL inhibition by suppressing MCL-1. *Cancer Discov,* 2014, vol. 4 (1), 42-52

- **FEARON, E. R.** Molecular genetics of colorectal cancer. *Annu Rev Pathol,* 2011, vol. 6, 479-507
- **HORN, T. ; T. SANDMANN ; B. FISCHER ; E. AXELSSON ; W. HUBER ; M. BOUTROS.** Mapping of signaling networks through synthetic genetic interaction analysis by RNAi. *Nat Methods,* 2011, vol. 8 (4), 341-346
- **JI, Z. ; C. N. NJAUW ; M. TAYLOR ; V. NEEL ; K. T. FLAHERTY ; H. TSAO.** p53 rescue through HDM2 antagonism suppresses melanoma growth and potentiates MEK inhibition. *J Invest Dermatol,* 2012, vol. 132 (2), 356-364
- **KATAYAMA, R. ; A. T. SHAW ; T. M. KHAN ; M. MINO-KENUDSON ; B. J. SOLOMON ; B. HALMOS ; N. A. JESSOP ; J. C. WAIN ; A. T. YEO ; C. BENES.** Mechanisms of acquired crizotinib resistance in ALK-rearranged lung Cancers. *Sci Transl Med,* 2012, vol. 4 (120), 120-117
- **LEHAR, J. ; A. KRUEGER ; G. ZIMMERMANN ; A. BORISY.** High-order combination effects and biological robustness. *Mol Syst Biol,* 2008, vol. 4, 215
- **LITO, P. ; N. ROSEN ; D. B. SOLIT.** Tumor adaptation and resistance to RAF inhibitors. *Nat Med,* 2013, vol. 19 (11), 1401-1409
- **PAU, G. ; F. FUCHS ; O. SKLYAR ; M. BOUTROS ; W. HUBER.** EBImage--an R package for image processing with applications to cellular phenotypes. *Bioinformatics,* 2010, vol. 26 (7), 979-981
- **PORTER, K. ; A. BABIKER ; K. BHASKARAN ; J. DARBYSHIRE ; P. PEZZOTTI ; K. PORTER ; A. S. WALKER ; C. COLLABORATION.** Determinants of survival following HIV-1 seroconversion after the introduction of HAART. *Lancet,* 2003, vol. 362 (9392), 1267-1274
- **ROBERT, C. ; B. KARASZEWSKA ; J. SCHACHTER ; P. RUTKOWSKI ; A. MACKIEWICZ ; D. STROIAKOVSKI ; M. LICHINITSER ; R. DUMMER ; F. GRANGE ; L. MORTIER.** Improved overall survival in melanoma with combined dabrafenib and trametinib. *N Engl J Med,* 2015, vol. 372 (1), 30-39
- **SAFAEE ARDEKANI, G. ; S. M. JAFARNEJAD ; L. TAN ; A. SAEEDI ; G. LI.** The prognostic value of BRAF mutation in colorectal cancer and melanoma: a systematic review and meta-analysis. *PLoS One,* 2012, vol. 7 (10), e47054
- **SHOEMAKER, R. H.** The NCI60 human tumour cell line anticancer drug screen. *Nat Rev Cancer,* 2006, vol. 6 (10), 813-823
- **SOLIT, D. B. ; N. ROSEN.** Towards a unified model of RAF inhibitor resistance. *Cancer Discov,* 2014, vol. 4 (1), 27-30
- **SULLIVAN, R. J. ; K. T. FLAHERTY.** Resistance to BRAF-targeted therapy in melanoma. *Eur J Cancer,* 2013, vol. 49 (6), 1297-1304
- **TURNER, N. C. ; J. RO ; F. ANDRE ; S. LOI ; S. VERMA ; H. IWATA ; N. HARBECK ; S. LOIBL ; C. HUANG BARTLETT ; K. ZHANG.** Palbociclib in Hormone-Receptor-Positive Advanced Breast Cancer. *N Engl J Med.,* 2015